# DEMANDE DE BREVET EUROPEEN

(11) **EP 1 535 905 A1**
(43) Date de publication de la demande: **01.06.2005**
(21) Numéro de dépôt: 04106117.7
(22) Date de dépôt: 26.11.2004
(51) Int. Cl.: C07D 207/14, C07D 401/04, C07D 207/12

(54) **Procédé de préparation de dérivés de paraphénylènediamine à groupement pyrrolidinyle, substitués par un radical azoté, et composés intermédiaires**

(30) Priorité: 28.11.2003 FR 0350939
(71) Demandeur: L'OREAL, 75008 Paris (FR)
(72) Inventeur: Bordier, Thierry, 93290 Tremblay en France (FR); Xu, Jinzhu, 75014 Paris (FR)
(74) Mandataire: Poulin, Gérard

(57) **Abrégé**

Procédé de synthèse de composés dérivés de paraphénylènediamine à groupement pyrolidinyle, substitués par un radical azoté, répondant à la formule (I) suivante :

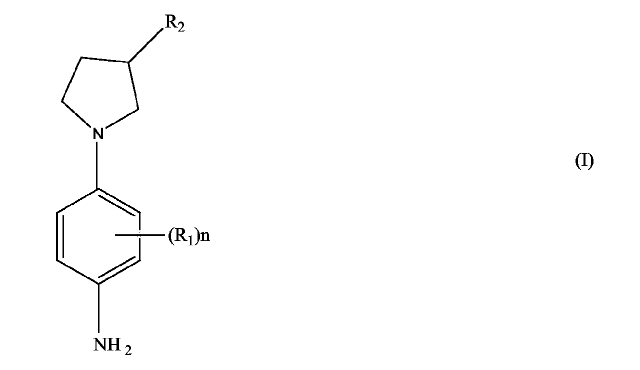

Composés intermédiaires à groupement pyrrolidinyle portant un radical azoté.

## Description

L'invention concerne un procédé de synthèse, de préparation de dérivés de paraphénylènediamine à groupement pyrrolidinyle, substitués par un radical azoté.

Elle a de même pour objet des composés intermédiaires à groupement pyrrolidinyle portant un radical azoté.

Les dérivés de paraphénylène diamine à groupement pyrrolidinyle qui peuvent être préparés par le procédé de l'invention, répondent par exemple à la formule (I) suivante : 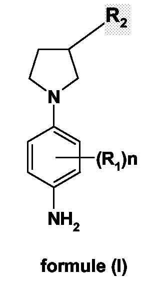 dans laquelle :
- le carbone portant le substituant R₂ sur le cycle pyrrolidine est racémique ou chiral ;
- n est un nombre entier de 0 à 4, étant entendu que lorsque n est supérieur ou égal à 2, alors les radicaux R₁ peuvent être identiques ou différents ;
- R₁ représente un atome d'halogène; une chaîne hydrocarbonée aliphatique en C₁ à C₆ ou alicyclique en C₃ à C₈, saturée ou insaturée ; un ou plusieurs atomes de carbone de la chaîne pouvant être remplacé(s) par un atome d'oxygène, d'azote, de silicium, de soufre, ou par un groupement SO₂ ; le radical R₁ ne comportant pas de liaison peroxyde, ni de radicaux diazo, nitro ou nitroso ;
- R₂ représente un radical azoté cationique
ou non.

Les composés dérivés, de paraphénylènediamine à groupement pyrolidinyle ; substitués par un radical azoté, de formule (I), sont utilisés comme base d'oxydation pour la teinture et dans les kits de teinture capillaires et ont l'avantage de présenter un bon profil toxicologique.

Certains des composés de la formule (I) ont été synthétisés.

Ainsi, le document WO-A-0168043 décrit quelques exemples de synthèse de certains composés entrant dans le cadre de la formule (I) qui utilisent une réaction de condensation du parafluoronitrobenzène avec une pyrrolidine déjà substituée par un groupement azoté (schéma 1).

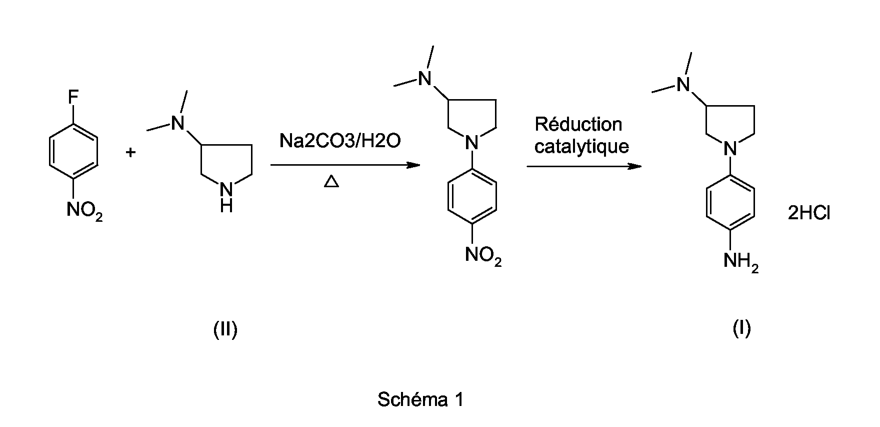

Or, les dérivés de pyrrolidine du type mis en oeuvre comme composé de départ dans le schéma 1, tels que la 3-(N,N-diméthylamino-)-pyrrolidine sont peu disponibles et chers. Leur préparation n'est pas aisée et demande une mise au point laborieuse. Il se pose donc un problème de fabrication à l'échelle industrielle de ces composés et par voie de conséquence des composés de formule (I).

Dans le document WO-A-0245675, des dérivés cationiques rentrant dans le cadre de la formule (I) ont été préparés selon le schéma 2 ci-après à partir d'un précurseur de formule (III) qui possède déjà le groupement pyrrolidinyle substitué par un groupement amine.

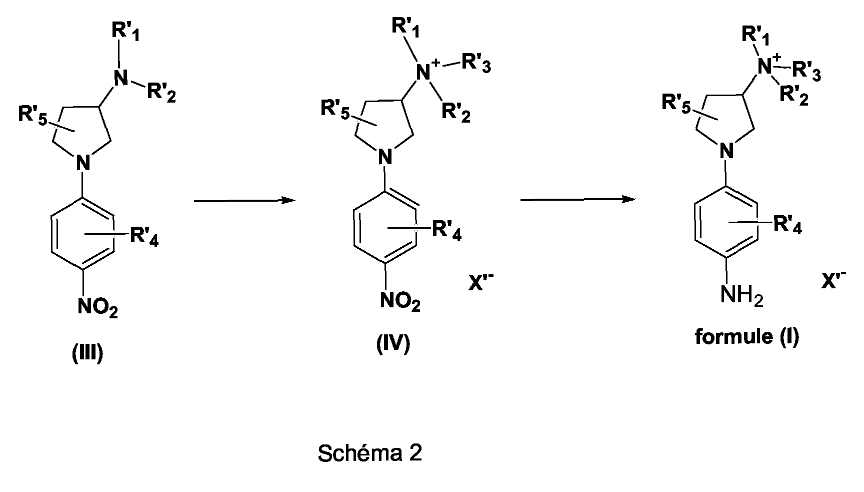

Les matières premières de la synthèse selon le schéma 2, c'est-à-dire les composés de la formule (III) ne sont pas commerciaux et n'ont pas d'accès chimique aisé. Les paraphénylènes diamines finales sont donc soit chères, soit non industrielles.

Il est par ailleurs possible de réaliser la synthèse d'une paraphenylènediamine à groupement pyrrolidinyle substitué par un radical imidazolinium, qui est un composé entrant dans le cadre de la formule (I), à partir d'un alcool de formule (V). Cet alcool est obtenu par une réaction de substitution de 3-pyrrolidinol avec le parafluoronitrobenzène. L'alcool est ensuite transformé en dérivé méthanesulfonate puis condensé avec la N-méthylimidazole pour fournir l'imidazolinium de formule (VI) (schéma 3).

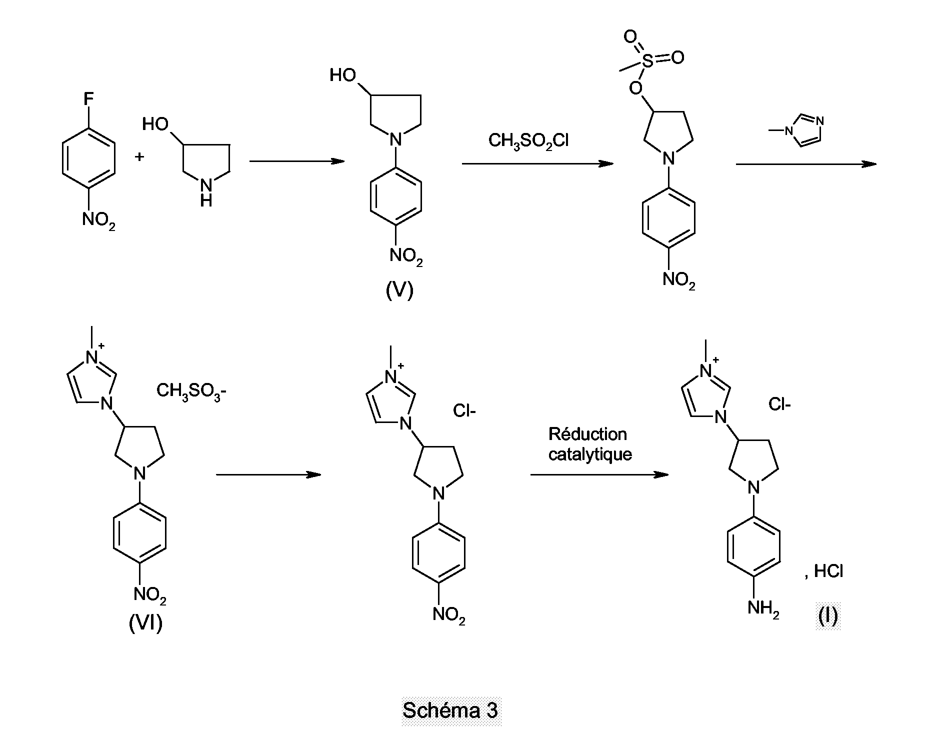

L'inconvénient de cette synthèse est que le 3-pyrrolidinol est une matière première chère et peu disponible industriellement.

Tous les procédés de préparation mentionnés ci-dessus, mettent en oeuvre des procédés de synthèse utilisant des matières premières ou des produits de départ peu disponible ou chers.

Il ressort de ce qui précède qu'il existe un besoin pour un procédé de préparation de dérivés ou composés de paraphénylènediamine à groupement pyrrolidinyle, substitués par un radical azoté, répondant notamment à la formule (I) ci-dessus, qui soit facile à mettre en oeuvre, reproductible, industriellement viable.

Il existe encore un besoin pour un procédé de préparation de ces composés, dérivés, qui utilise des matières premières ou produits de départ facilement disponibles et d'un faible coût et qui permette d'obtenir les composés finaux paraphénylène diamines à groupement pyrrolidinyle avec un bon rendement.

Il existe en particulier un besoin pour un tel procédé qui évite notamment d'utiliser en tant que matières premières les précurseurs des formules (II), (III) ou le 3-pyrrolidinol qui sont des composés chers et/ou peu ou pas disponibles sur un plan industriel.

Le but de la présente invention est de fournir un procédé de synthèse, de composés dérivés de paraphénylènediamines à groupement pyrrolidinyle, substitués par un radical azoté, qui réponde entre autres aux besoins énumérés plus haut.

Le but de la présente invention est encore de fournir un procédé de préparation de composés dérivés de paraphénylènediamine à groupement pyrolidinyle, substitués par un radical azoté qui ne présente pas les inconvénients, défauts, limitations et désavantages des procédés de l'art antérieur et qui résolve les problèmes des procédés de l'art antérieur.

Ce but et d'autres encore sont atteints conformément à l'invention, par un procédé de synthèse, de composés dérivés de paraphénylènediamine à groupement pyrolidinyle, substitués par un radical azoté, répondant à la formule (I) suivante : 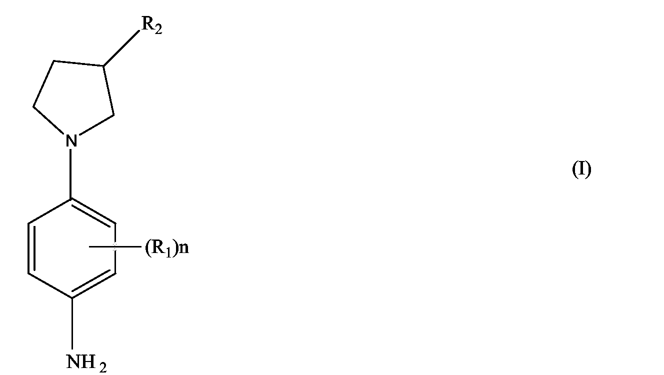 dans laquelle :
- le carbone portant le substituant R₂ sur le cycle pyrrolidine est racémique ou chiral, c'est-à-dire de configuration (R) ou (S) ;
- n est un nombre entier de 0 à 4, étant entendu que lorsque n est supérieur ou égal à 2, alors les radicaux R₁ peuvent être identiques ou différents ;
- R₁ représente un atome d'halogène; une chaîne hydrocarbonée aliphatique linéaire ou ramifié en C₁-C₆, ou alicyclique en C₃-C₈, saturée ou insaturée, un radical aryle, un radical arylalkyle dont la chaîne alkyle est en C₁-C₆, un ou plusieurs atomes de carbone de la chaîne hydrocarbonée et de la chaîne alkyle du radical arylalkyle pouvant être remplacés par un atome d'oxygène, d'azote, de silicium, de soufre, ou par un groupement SO₂ ; le radical R₁ ne comportant pas de liaison peroxyde, ni de radicaux diazo, nitro ou nitroso ;
- R₂ représente un radical azoté cationique ou non ;
   dans lequel on réalise les étapes successives suivantes :
   a) on condense une aniline parasubstituée de formule (VII) : 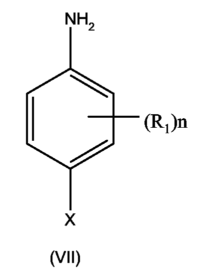 dans laquelle R₁ et n ont la signification déjà donnée plus haut pour la formule (I), et X représente un groupement précurseur d'une fonction amine , avec un dérivé de formule (VIII) : 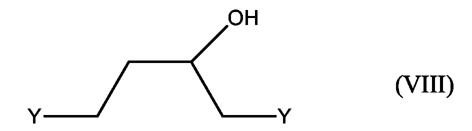 dans laquelle Y représente un groupe partant, pour former un composé de formule (IX) : 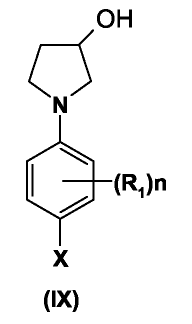 dans laquelle X₁, R₁, et n ont la signification déjà donnée plus haut ;
   b) on active le composé de formule (IX) pour former un composé de formule (X) : 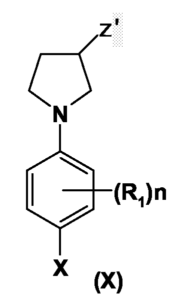 dans laquelle X, R₁ et n ont la signification déjà donnée plus haut et Z' représente un groupe partant ;
   c) on fait réagir le composé de formule (X) avec l'ammoniaque ou une amine primaire, secondaire ou tertiaire, ou avec un composé portant un hétérocycle aromatique azoté ;
      pour former un composé de formule (XI) : 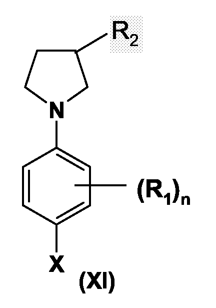 dans laquelle X, R₁, R₂, et n ont la signification déjà donnée plus haut ;
   d) on transforme le groupement X en fonction amine, pour former le composé de formule (I).

Les différentes étapes du procédé selon l'invention sont illustrées de manière simplifiée sur schéma 5 donné plus loin.

Le procédé selon l'invention fait appel à des produits de départ (VII) et (VIII) qui sont facilement disponibles industriellement, et d'un faible coût. Par exemple, le 1,4-dibromobutanol entrant dans la formule (VIII), peut-être facilement préparé à partir de 1,2,4-butanetriol selon le document JP-A-02 264735.

Le procédé selon l'invention ne fait pas appel, au contraire des procédés de l'art antérieur, à l'utilisation de précurseurs des formules (II) et (III) ou de 3-pyrolidinols en tant que matières premières, de ce fait un des inconvénients majeurs des procédés de l'art antérieur est évité.

Le procédé selon l'invention est un procédé facile à mettre en oeuvre, reproductible et industriellement viable.

L'invention a de plus pour objet, en tant que composés nouveaux, certains des composés intermédiaires de formule (IX) et de formule (XI) données précédemment dans le cadre de la description du procédé. Ces composés nouveaux, sont les composés qui répondent à la formule (IX) ou à la formule (XI) mentionnées plus haut dans lesquelles le groupement X représente un groupement amide, carbamate, un groupement nitro (dans le cas des composés de formule (IX)) ou un groupement nitroso, à savoir X représente plus particulièrement un groupement -NHCOR, -NHCOOR, un groupement nitroso ou un groupement nitro (dans le cas des composés de formule (IX)) où R représentent une chaîne aliphatique, linéaire ou ramifiée, saturée ou non, de préférence saturée, en C₁-C₆, un groupement aryle ou un groupement arylalkyle dont la partie alkyle est en C₁-C₆ ; exceptés (outre les composés où X = nitro dans la formule (XI)) certains composés particuliers énumérés plus loin.

Il est à noter que dans le cadre de la définition des composés (IX) et (XI) donnée en relation avec le procédé dans lequel ils sont mis en oeuvre, aucun composé particulier n'est exclu et X peut être NO₂ dans la formule (XI).

L'invention va maintenant être décrite de manière détaillée dans ce qui suit :

Dans le cadre de l'invention, une chaîne hydrocarbonée aliphatique est une chaîne linéaire ou ramifiée pouvant contenir des insaturations du type alcène ou alcyne. Une chaîne hydrocarbonée alicyclique est une chaîne cyclique, saturée ou insaturée ne contenant pas de structure cyclique aromatique.

Lorsque la chaîne est interrompue par un atome Y' d'oxygène, de soufre, d'azote, de silicium ou SO₂, on obtient, par exemple, un motif CH₂-Y'-CH₂.

Dans la formule (I), n peut être égal à 0 et le cycle benzénique ne porte alors pas de substituant. Dans le cas contraire, (n différent de 0 et par exemple égal à 1) et à titre d'exemple, R₁ peut être un atome de chlore, un radical méthyle, éthyle, isopropyle, hydroxyméthyle méthoxyméthyle, hydroxyéthyle, 2-hydroxyéthyle, 3-hydroxypropyle, 1,2-dihydroxyéthyle, méthoxy, éthoxy, 2-hydroxyéthyloxy, phényle.

Comme mentionné auparavant, dans la formule (I), lorsque n est différent de zéro et par exemple égal à 1, R₁ est un atome d'halogène ; une chaîne hydrocarbonée aliphatique, linéaire ou ramifiée en C₁ à C₆, ou alicyclique en C₃ à C₈, saturée ou insaturée, un radical aryle, un radical arylalkyle dont la chaîne alkyle est en C₁-C₆, un ou plusieurs atomes de carbone de la chaîne hydrocarbonée et de la chaîne alkyle du radical arylalkyle pouvant être remplacés par un atome d'oxygène, d'azote, de silicium, de soufre, ou par un groupement SO₂ ; le radical R₁ ne comportant pas de liaison peroxyde, ni de radicaux diazo, nitro ou nitroso.

De préférence, lorsque n est différent de 0, et par exemple égal à 1, R₁ est choisi parmi le chlore, le brome, un radical alkyle en C₁-C₄, hydroxyalkyle en C₁-C₄, aminoalkyle en C₁-C₄, alcoxy en C₁-C₄, hydroxyalcoxy en C₁-C₄.

A titre d'exemples préférés, R₁ est choisi parmi un radical méthyle, hydroxyméthyle, 2-hydroxyéthyle, 1,2-dihydroxyéthyle, méthoxy, isopropyloxy, 2-hydroxyéthoxy.

Comme indiqué auparavant, le radical R₂ représente un radical azoté cationique ou non cationique.

Si R₂ est un radical azoté cationique, il représente plus particulièrement un radical onium comme par exemple un radical ammonium, imidazolium, ou pyridinium.

Si R₂ est un radical azoté non cationique, il représente plus particulièrement un radical amine primaire (-NH₂), secondaire (-NHR) ou tertiaire (-NR₂) où R, identiques ou non, représentent un radical aliphatique, linéaire ou ramifié, saturé ou non, en C₁-C₂₂ , de préférence un radical alkyle en C₁-C₂₂ ; un radical alicyclique, saturé ou non, en C₃-C₈ ; un radical monohydroxyalkyle en C₁-C₂₂, de préférence en C₁-C₆ ; un radical polyhydroxyalkyle en C₂-C₂₂, de préférence en C₂-C₆ ; un radical alcoxy (C₁-C₆) alkyle en C₁-C₂₂, de préférence en C₁-C₆ ; un radical aryle ; un radical arylalkyle dont la partie alkyle est en C₁-C₆, comme le radical benzyle par exemple ; un radical amidoalkyle en C₁-C₆ ; un radical trialkyl (C₁-C₆) silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl (C₁-C₆) carbonyle, amido ou alkyl (C₁-C₆) sulfonyle.

Par ailleurs, les radicaux R peuvent former deux à deux, ensemble avec l'atome d'azote auxquels ils sont rattachés, un cycle saturé carboné comprenant 3 à 9 chaînons, de préférence 4, 5, 6, 7 ou 8 chaînons, pouvant contenir un ou plusieurs hétéroatomes. A titre d'exemples de tels cycles, on peut citer les cycles azétidine, pyrrolidine, pipéridine, pipérazine, morpholine, ledit hétérocycle pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical carboxyle, un radical alkyl(C₁-C₆)carbonyle, un radical thio (-SH), un radical thioalkyle (-R-SH) en C₁-C₆, un radical alkyl(C₁-C₆)thio, un radical amino, un radical amino mono ou di-substitué par un radical alkyl(C₁-C₆), alkyl(C₁-C₆)carbonyle, amido ou alkyl(C₁-C₆)sulfonyle.

Conformément à un autre mode de réalisation, R₂ est un radical dérivé de l'amino-guanidine (de formule -NH-NH-C (NH₂) =NH).

Si le radical R₂ de la formule (I) (et (XI)) est un radical onium Z, il correspond dans un premier mode de réalisation à la formule (XIII) : 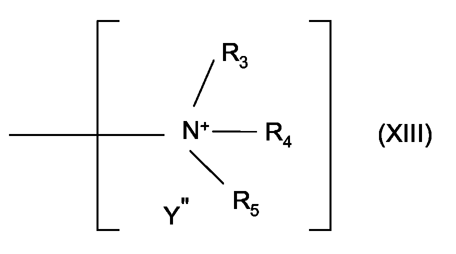 dans laquelle :
- R₃, R₄ et R₅, pris séparément, identiques ou non, représentent un atome d'hydrogène, un radical aliphatique, linéaire ou ramifié, saturé ou non, en C₁-C₂₂, de préférence un radical alkyle en C₁-C₂₂ ; un radical alicyclique, saturé ou non, en C₃-C₈ ; un radical monohydroxyalkyle en C₁-C₂₂, de préférence en C₁-C₆ ; un radical polyhydroxyalkyle en C₂-C₂₂, de préférence en C₂-C₆ ; un radical alcoxy (C₁-C₆) alkyle en C₁-C₂₂, de préférence en C₁-C₆ ; un radical aryle ; un radical arylalkyle dont la partie alkyle est en C₁-C₆, comme le radical benzyle par exemple ; un radical amidoalkyle en C₁-C₆ ; un radical trialkyl (C₁-C₆) silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl (C₁-C₆) carbonyle, amido ou alkyl (C₁-C₆) sulfonyle ;
- R₃, R₄ et R₅ ensemble, deux à deux, forment, avec l'atome d'azote auxquels ils sont rattachés, un cycle saturé carboné comprenant 3 à 9 chaînons, de préférence 4, 5, 6, 7 ou 8 chaînons, pouvant contenir un ou plusieurs hétéroatomes. A titre d'exemples, on peut citer les cycles azétidine, pyrrolidine, pipéridine, pipérazine ou morpholine, ledit hétérocycle cationique pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆, un radical amido, un radical carboxyle, un radical alkyl(C₁-C₆)carbonyl, un radical thio (-SH), un radical thioalkyle (-R-SH) en C₁-C₆, un radical alkyl(C₁-C₆)thio, un radical amino, un radical amino mono ou di-substitué par un radical alkyl (C₁-C₆), alkyl (C₁-C₆) carbonyle, amido ou alkyl (C₁-C₆) sulfonyle ;
- Y" est un contre ion.

Dans la formule (XIII), selon un mode plus particulier de réalisation, R₃, R₄ et R₅ séparément sont choisis de préférence parmi un radical alkyle en C₁-C₆, un radical monohydroxyalkyle en C₁-C₄, un radical polyhydroxyalkyle en C₂-C₄, un radical alcoxy (C₁-C₆)alkyle en C₁-C₄, un radical trialkyl (C₁-C₆) silanealkyle en C₁-C₆ , ou R₃ avec R₄ forment ensemble un cycle azétidine, un cycle pyrrolidine, pipéridine, pipérazine, morpholine, R₅ étant choisi dans ce cas parmi un radical alkyle en C₁-C₆ ; un radical monohydroxyalkyle en C₁-C₆ ; un radical polyhydroxyalkyle en C₂-C₆.

Lorsque le radical R₂ correspond à la formule (XIII), il est de préférence un radical trialkylammonium dont les radicaux alkyles peuvent être substitués.

Selon un deuxième mode de réalisation, le radical R₂ représente le radical onium Z correspondant à la formule (XIV) : 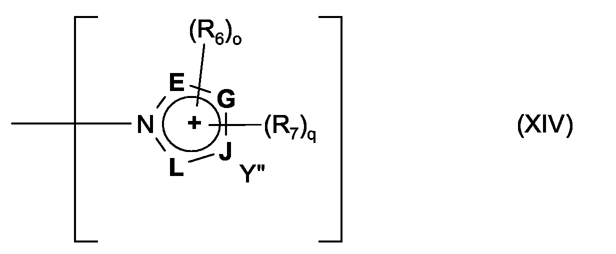 dans laquelle :
- les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique ;
- q est un nombre entier compris entre 0 et 4 inclus ;
- o est un nombre entier compris entre 0 et 3 inclus ;
- q+o est un nombre entier compris entre 0 et 4 inclus ;
- R₇, identiques ou différents, représentent un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆ ; étant entendu que les radicaux R₇ sont portés par un atome de carbone ;
- R₆, identique ou différent, représente un radical alkyle en C₁-C₆, un radical monohydroxyalkyle en C₁-C₆, un radical polyhydroxyalkyle en C₂-C₆, un radical trialkyl (C₁-C₆) silanealkyle en C₁-C₆, un radical alcoxy (C₁-C₆) alkyle en C₁-C₆, un radical carbamylalkyle C₁-C₆, un radical alkyl (C₁-C₆) carboxyalkyle en C₁-C₆, un radical benzyle ; étant entendu que les radicaux R₆ sont portés par un azote ;
- Y" est un contre-ion.
   A titre d'exemple, les sommets E, G, J et L peuvent former un cycle pyrrolique, imidazolique, pyrazolique, oxazolique, thiazolique ou triazolique, de préférence imidazolique.

Selon un troisième mode de réalisation, R₂ représente le radical onium Z correspondant à la formule (XV) : 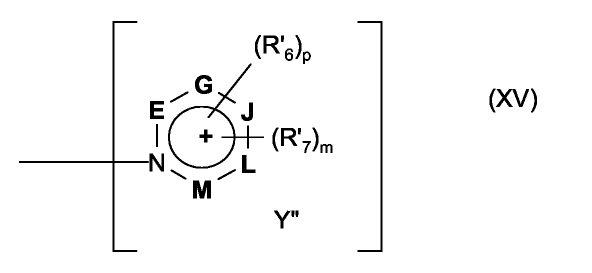 dans laquelle :
- les sommets E, G, J, L et M identiques ou différents, représentent un atome de carbone, d'oxygène, de soufre ou d'azote former un cycle choisi parmi les cycles pyridiniques, pyrimidiniques, pyraziniques, triaziniques et pyridaziniques ;
- p est un nombre entier compris entre 0 et 3 inclus ;
- m est un nombre entier compris entre 0 et 5 inclus ;
- p+m est un nombre entier compris entre 0 et 5 inclus ;
- R'₇, identiques ou différents, représentent un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆ ; étant entendu que les radicaux R'₇ sont portés par un atome de carbone ;
- R'₆, identique ou différent, représente un radical alkyle en C₁-C₆, un radical monohydroxyalkyle en C₁-C₆, un radical polyhydroxyalkyle en C₂-C₆, un radical trialkyl (C₁-C₆) silanealkyle en C₁-C₆, un radical alcoxy (C₁-C₆) alkyle en C₁-C₆, un radical carbamylalkyle en C₁-C₆, un radical alkyl (C₁-C₆) carboxyalkyle en C₁-C₆, un radical benzyle ; étant entendu que les radicaux R'₆ sont portés par un azote ;
- Y" représente un contre-ion.
   De préférence, les sommets E, G, J, L et M forment avec l'azote du cycle un cycle pyridinique ou pyrimidinique.

De préférence, R₇, R₆, R'₇, et R'₆ sont des radicaux alkyles pouvant être substitués.

Dans le cadre de l'invention, le contre ion (Y") peut être choisi parmi un atome d'halogène tel que le brome, le chlore, le fluor ou l'iode, un hydroxyde, un citrate, un succinate, un tartrate, un lactate, un tosylate, un mésylate, un benzènesulfonate, un acétate, un hydrogènesulfate ou un alkylsulfate en C₁-C₆ tel que par exemple le méthylsulfate, ou l'éthylsulfate.

De préférence, le contre ion (Y") est choisi parmi un atome d'halogène tel que le brome, le chlore ou l'iode, un tosylate, un mésylate, un benzènesulfonate, ou un alkylsulfate en C₁-C₆ tel que par exemple le méthylsulfate, ou l'éthylsulfate.

Des exemples de composés de formule (I) qui peuvent être préparés par le procédé de l'invention sont les suivants :

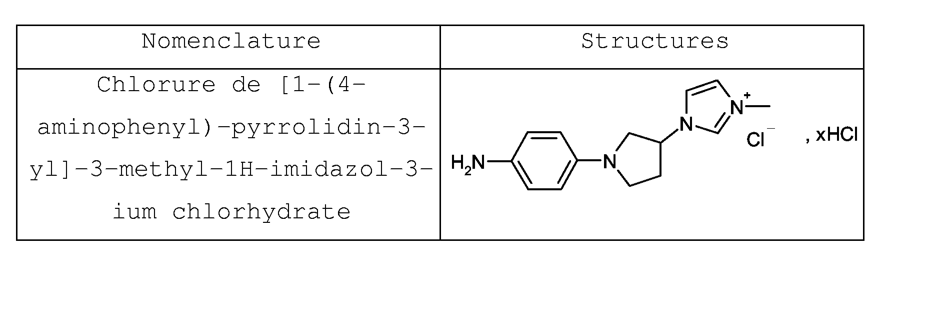

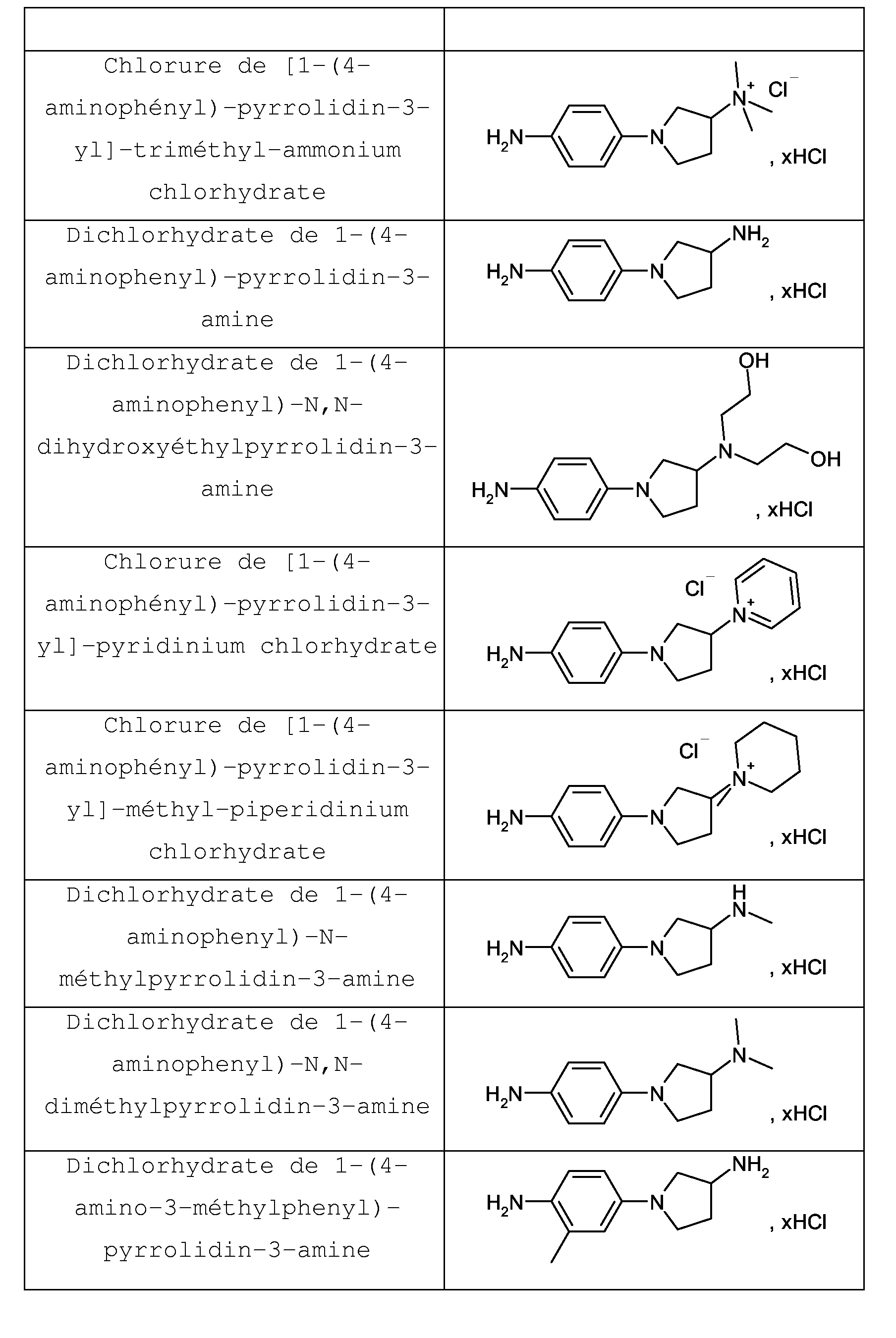

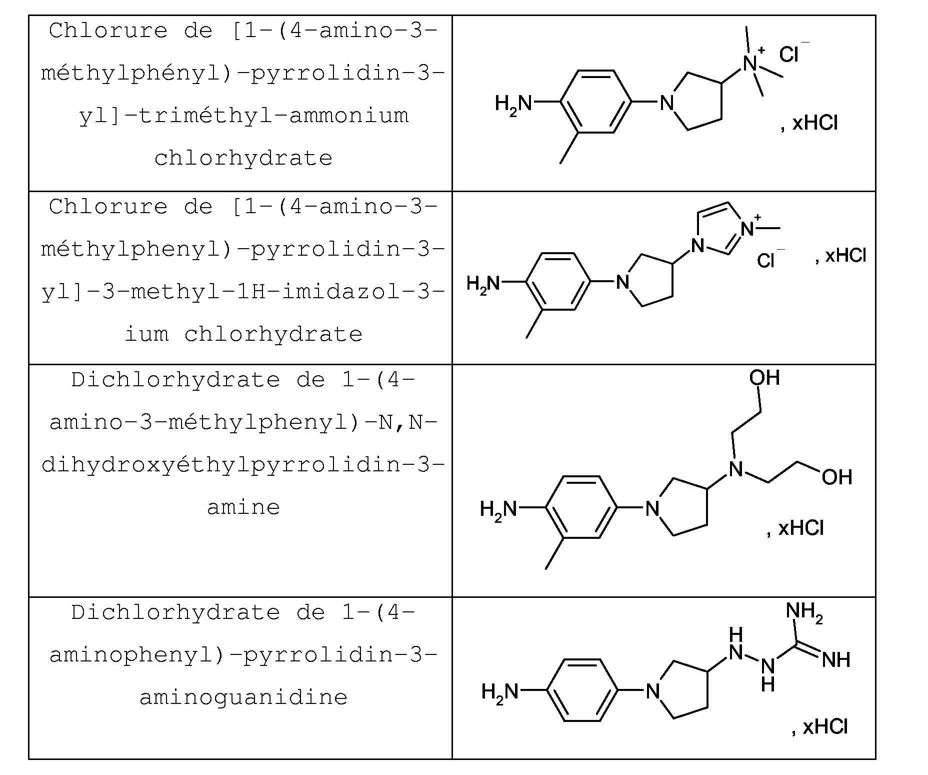

Les différentes étapes du procédé de l'invention sont illustrées sur le schéma 5 suivant :

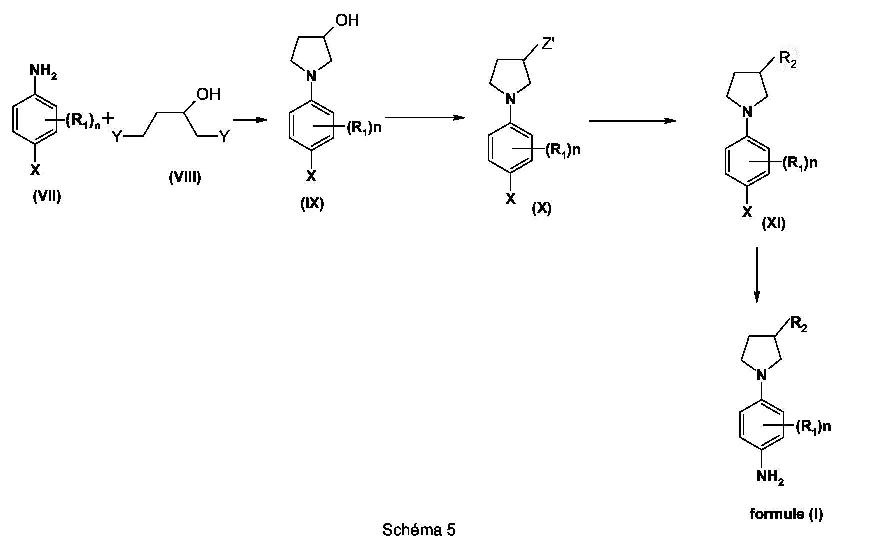

Bien que cela n'apparaisse pas sur le schéma ci-dessus, et qu'il n'y sera pas fait référence dans la description détaillée du procédé selon l'invention, il est à noter que le procédé peut éventuellement comprendre, à chaque fois que cela est nécessaire, au moins une étape intermédiaire de protection et de déprotection d'un groupement particulier présent sur une molécule que l'on fait réagir. Ce type d'étape fait partie des connaissances générales de l'homme du métier qui est à même de déterminer s'il est nécessaire de la mettre en oeuvre ainsi que les moyens pour le faire, en fonction du groupement à protéger et de la réaction ultérieure effectuée.

La première étape du procédé de l'invention est une étape dans laquelle on condense une aniline parasubstituée de formule (VII) : 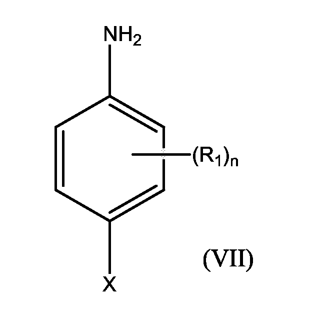 dans laquelle R₁ et n ont la signification déjà donnée ci-dessus pour la formule (I) et X représente un groupement précurseur d'une fonction amine, à titre d'exemples de groupements de ce type, on peut notamment citer les groupements nitro, -NHCOR, -NHCOOR, où R représentent une chaîne aliphatique, linéaire ou ramifiée, saturée ou non, de préférence saturée, en C₁ à C₆, un groupement aryle ou groupement arylalkyle dont la partie alkyle est en C₁-C₆, avec un dérivé de formule (VIII) : 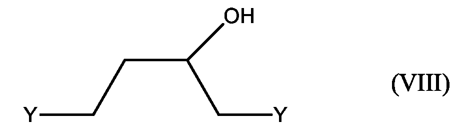 dans laquelle Y représente groupement partant pour former un composé de formule (IX) :

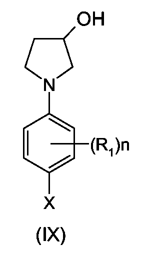

Dans laquelle R₁, X, et n ont les significations (et les significations préférées) déjà données plus haut.

Par groupement partant, on entend de manière classique, tout radical susceptible d'être éliminé au cours d'une réaction chimique. Dans le cas présent, et à titre d'exemples, ce groupe est plus particulièrement choisi parmi un halogène, comme Cl, Br ou I ; un radical -OSO₂R' ou -OSO₃R', où R' représente un radical aliphatique, linéaire ou ramifié, saturé ou non, en C₁-C₆, aryle, arylalkyle dont la partie alkyle est en C₁-C₆. De préférence, R' représente un radical méthyle, paraméthylphényle, éthyle, phényle, benzyle.

Des composés préférés de formule (VIII) sont choisis parmi le dibromobutanol, le ditosylbutanol, le dimésylbutanol. Des composés préférés de formule (VII) sont choisis parmi le para-acétamidoaniline, le paraacétamido-méta-méthylaniline.

La condensation de l'aniline parasubstituée de formule (VII) sur le composés de formule (VIII) est réalisée de manière connue de l'homme du métier. A titre d'exemple, on peut procéder en chauffant entre 30° et 150°C le mélange de l'aniline (VII) et du composé de formule (VIII) pendant 1 heure à 24 heures dans un solvant, de préférence tel que l'éthanol, le diméthylformamide, l'isopropanol, le toluène, le tétrahydrofurane, le méthyltertbutyl éther, l'acétate d'isopropyle. Habituellement, cette réaction est effectuée en présence d'une base telle que, par exemple, CH₃COONa, Na₂CO₃, K₂CO₃.

Le composé (IX) qui se présente généralement sous forme solide après traitement du mélange réactionnel avec l'eau est séparé, par exemple par filtration, et utilisé pour la deuxième étape b) du procédé selon l'invention.

Dans l'étape b) suivante, on réalise l'activation du composé de formule (IX) ci-dessus pour former un composé de formule (X) : 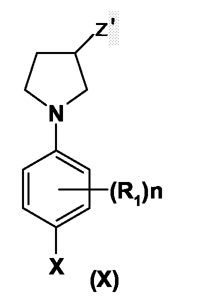 dans laquelle X, R₁ et n ont la signification (et les significations préférées) déjà donnée plus haut et Z' représente un groupe partant. La définition indiquée ci-dessus pour Y reste valable dans le cas de Z' et l'on pourra s'y référer.

La réaction d'activation du composé de formule (IX) peut être réalisée, par exemple, par un traitement avec un halogénure de sulfonyle dans la pyridine. Il est de même envisageable de mettre en oeuvre un solvant inerte, comme notamment le dichlorométhane, le 1,2-dichloroéthane, le toluène, le tétrahydrofurane, l'acétate d'éthyle, en présence d'une base organique ou minérale du type triéthylamine ou carbonate de sodium. Le réactif d'activation préféré est le chlorure de méthanesulfonyle ou le chlorure de toluènesulfonyle qui donne un groupe Z' qui est un groupe MeSO₃- ou C₇H₇SO₃-sur le cycle pyrrolidinyle.

Le composé (X), qui se présente généralement sous forme solide, est séparé du mélange réactionnel, par exemple, par filtration, après un traitement avec l'eau et utilisé pour la troisième étape c) du procédé de l'invention.

Dans l'étape c) suivante, on fait réagir le composé de formule (X) avec de l'ammoniaque ou une amine primaire, secondaire ou tertiaire, ou encore avec un composé portant un hétérocycle azoté aromatique pour former un composé de formule (XI) : 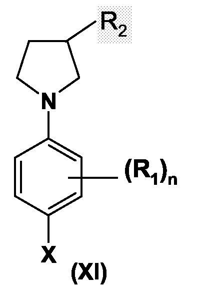 dans laquelle X, R₁, R₂ et n ont la signification (et les significations préférées) déjà données plus haut.

En ce qui concerne l'amine primaire, secondaire ou tertiaire, on peut utiliser un composé de formule suivante : 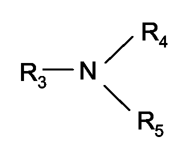 dans laquelle :
R₃, R₄ et R₅, pris séparément, identiques ou non, représentent un hydrogène, un radical aliphatique, linéaire ou ramifié, saturé ou non, en C₁-C₂₂, de préférence un radical alkyle en C₁-C₂₂ ; un radical alicyclique, saturé ou non, en C₃-C₈ ; un radical monohydroxyalkyle en C₁-C₂₂, de préférence en C₁-C₆ ; un radical polyhydroxyalkyle en C₂-C₂₂, de préférence en C₂-C₆ ; un radical alcoxy (C₁-C₆) alkyle en C₁-C₂₂, de préférence en C₁-C₆ ; un radical aryle ; un radical arylalkyle dont la partie alkyle est en C₁-C₆, comme le radical benzyle par exemple ; un radical amidoalkyle en C₁-C₆ ; un radical trialkyl (C₁-C₆) silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl (C₁-C₆) carbonyle, amido ou alkyl (C₁-C₆) sulfonyle ;
ou R₃, R₄ et R₅ ensemble, deux à deux, forment, avec l'atome d'azote auxquels ils sont rattachés, un cycle saturé carboné comprenant 3 à 9 chaînons, de préférence 4, 5, 6, 7 ou 8 chaînons, pouvant contenir un ou plusieurs hétéroatomes. A titre d'exemples, on peut citer les cycles azétidine, pyrrolidine, pipéridine, pipérazine ou morpholine, ledit hétérocycle cationique pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆, un radical amido, un radical carboxyle, un radical alkyl(C₁-C₆)carbonyl, un radical thio (-SH), un radical thioalkyle (-R-SH) en C₁-C₆, un radical alkyl (C₁-C₆) thio, un radical amino, un radical amino mono ou di-substitué par un radical alkyl (C₁-C₆), alkyl (C₁-C₆) carbonyle, amido ou alkyl (C₁-C₆) sulfonyle.

Il est de même envisageable de mettre en oeuvre l'étape avec de l'amino-guanidine (H₂N-NH-C(NH₂)=NH).

En ce qui concerne le composé comprenant au moins un hétérocycle azoté aromatique, on peut citer les composés de formule suivante : 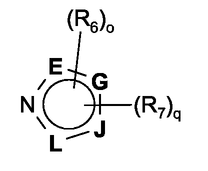 dans laquelle :
- les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique ;
- q est un nombre entier compris entre 0 et 4 inclus ;
- o est un nombre entier compris entre 0 et 3 inclus ;
- q+o est un nombre entier compris entre 0 et 4 inclus ;
- R₇, identiques ou différents, représentent un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆ ; étant entendu que les radicaux R₇ sont portés par un atome de carbone ;
- R₆ , identique ou différent, représente un radical alkyle en C₁-C₆, un radical monohydroxyalkyle en C₁-C₆, un radical polyhydroxyalkyle en C₂-C₆, un radical trialkyl (C₁-C₆) silanealkyle en C₁-C₆, un radical benzyle ; étant entendu que les radicaux R₆ sont portés par un azote.

A titre d'exemple, les sommets E, G, J et L peuvent former un cycle pyrrolique, imidazolique, pyrazolique, oxazolique, thiazolique et triazolique, de préférence imidazolique.

On peut aussi utiliser un composé de formule suivante : 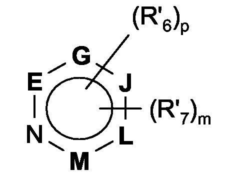 dans laquelle :
- les sommets E, G, J, L et M identiques ou différents, représentent un atome de carbone, d'oxygène, de soufre ou d'azote former un cycle choisi parmi les cycles pyridiniques, pyrimidiniques, pyraziniques, triaziniques et pyridaziniques ;
- p est un nombre entier compris entre 0 et 3 inclus ;
- m est un nombre entier compris entre 0 et 5 inclus ;
- p+m est un nombre entier compris entre 0 et 5 inclus ;
- R'₇, identiques ou différents, représentent un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆ ; étant entendu que les radicaux R'₇ sont portés par un atome de carbone ;
- R'₆, identique ou différent, représente un radical alkyle en C₁-C₆, un radical monohydroxyalkyle en C₁-C₆, un radical polyhydroxyalkyle en C₂-C₆, un radical trialkyl (C₁-C₆) silanealkyle en C₁-C₆, un radical alcoxy (C₁-C₆) alkyle en C₁-C₆, un radical carbamylalkyle en C₁-C₆, un radical alkyl (C₁-C₆) carboxyalkyle en C₁-C₆, un radical benzyle ; étant entendu que les radicaux R'₆ sont portés par un azote.

De préférence, les sommets E, G, J, L et M forment avec l'azote du cycle un cycle pyridinique et pyrimidinique.

Selon un mode de réalisation avantageux de l'invention, les amines primaires sont choisies parmi la méthylamine, l'éthylamine, la propylamine, la butylamine, l'isopropylamine, l'aniline, la benzylamine.

En ce qui concerne les amines secondaires, ces dernières sont de préférence choisies parmi la diméthylamine, la diéthylamine, la méthyléthylamine, la diisopropylamine,la dihydroxyéthylamine, la pyrolidine.

En ce qui concerne les amines tertiaires, elles sont avantageusement choisies parmi la triméthylamine, la triéthylamine, la diméthyléthylamine, la N-méthylpipéridine.

Quant aux composés portant un hétérocycle azoté aromatique, ils sont de préférence choisis parmi l'imidazole, le N-méthylimidazole, la pyridine.

Dans cette étape c), la réaction du composé de formule (X) avec une amine ou un composé portant un hétérocycle aromatique est réalisée de manière connue en soi. Plus particulièrement, à titre d'exemple, on peut effectuer la réaction en chauffant entre 50°C et 150°C, pendant 1 heure à 24 heures, dans un solvant tel que la méthylisobutyl cétone, la méthyléthylcétone, le tétrahydrofurane, l'isobutanol, le propanol, le tertbutylméthyléther, l'acétate d'isopropyle, le toluène, notamment.

Les produits obtenus dans l'étape c) du procédé de l'invention, sont généralement des produits solides qui peuvent être séparés du mélange réactionnel, par exemple par filtration, avant d'être utilisés dans l'étape d) du procédé de l'invention.

Dans l'étape d), on transforme le groupement X en fonction amine pour former le composé final recherché de formule (I).

Plus particulièrement, on procède à l'hydrolyse du composé de formule (XI) dans le cas où X représente un groupement -NHCOR ou -NHCOOR, définis auparavant.

Selon une autre possibilité, on procède à une réaction de réduction, telle que l'hydrogénation, du composé de formule (XI) dans le cas où X représente un groupement nitro.

A titre d'exemple non limitatif de mise en oeuvre, la réaction d'hydrolyse du composé de formule (XI) est réalisée dans un solvant tel que notamment l'eau, l'éthanol, l'isopropanol, en présence d'un acide minéral comme par exemple l'acide chlorhydrique, l'acide bromhydrique, l'acide sulfurique. Habituellement, la température de réaction est comprise entre 20°C et 120°C.

La réaction d'hydrogénation du composé de formule (XI) est généralement réalisée dans un solvant tel que le méthanol, l'éthanol, l'isopropanol, avantageusement en présence d'un catalyseur du type palladium sur charbon, sous une pression d'hydrogène comprise entre 1 et 50 bars à une température de 20°C à 120°C.

Un autre objet de l'invention est constitué par certains des composés intermédiaires de formule (IX) qui sont des composés nouveaux.

Il s'agit des composés de formule (IX) définis plus haut dans le cadre de la description du procédé mais à l'exclusion de certains composés spécifiques.

Ces composés intermédiaires nouveaux répondent à la formule : 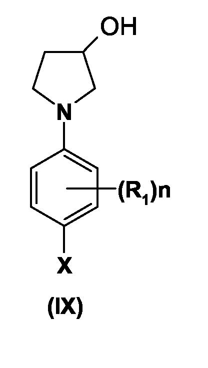 dans laquelle :
- le carbone portant le substituant hydroxyle sur le cycle pyrrolidine est racémique ou chiral ;
- n est un nombre entier de 0 à 4, étant entendu que lorsque n est supérieur ou égal à 2, alors les radicaux R₁ peuvent être identiques ou différents ;
- R₁ représente un atome d'halogène; une chaîne hydrocarbonée aliphatique en C₁-C₆ ou alicyclique en C₃-C₈, saturée ou insaturée, un radical aryle, un radical arylalkyle dont la partie alkyle est en C₁-C₆, un ou plusieurs atomes de carbone de la chaîne hydrocarbonée et la chaîne alkyle du radical arylalkyle pouvant être remplacés par un atome d'oxygène, d'azote, de silicium, de soufre, ou par un groupement SO₂ ; le radical R₁ ne comportant pas de liaison peroxyde, ni de radicaux diazo, nitro ou nitroso ;
- X représente un groupement -NHCOR, - NHCOOR, un groupement nitroso ou un groupement nitro où R représentent une chaîne aliphatique, linéaire ou ramifiée, saturée ou non, de préférence saturée, en C₁-C₆, un groupement aryle ou un groupement arylalkyle dont la partie alkyle est en C₁-C₆, exceptés les trois composés dans le tableau ci-dessous : 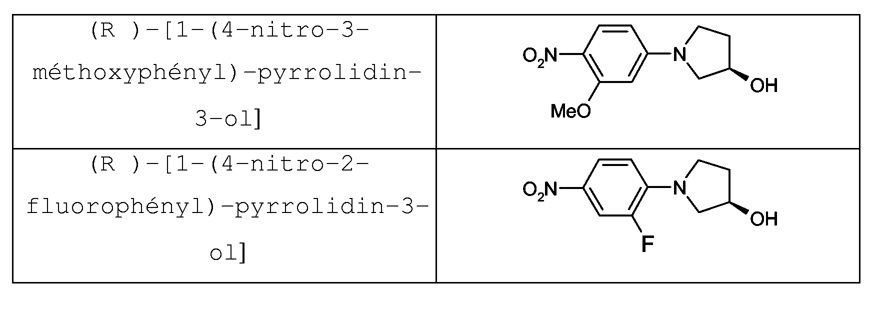 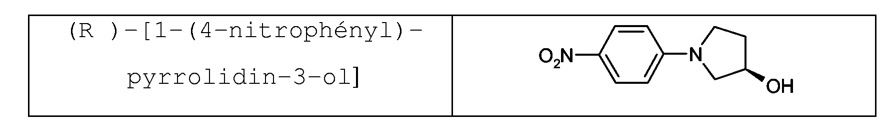 et exceptés les composés suivants :

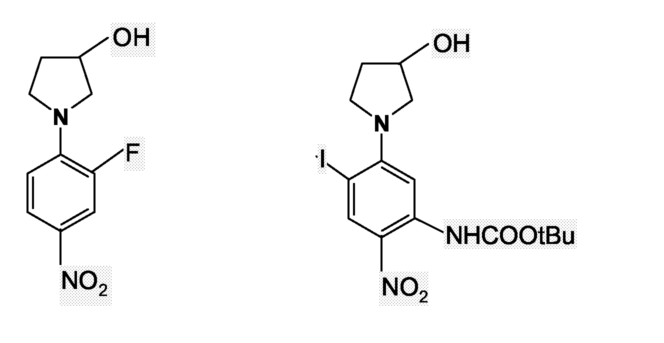

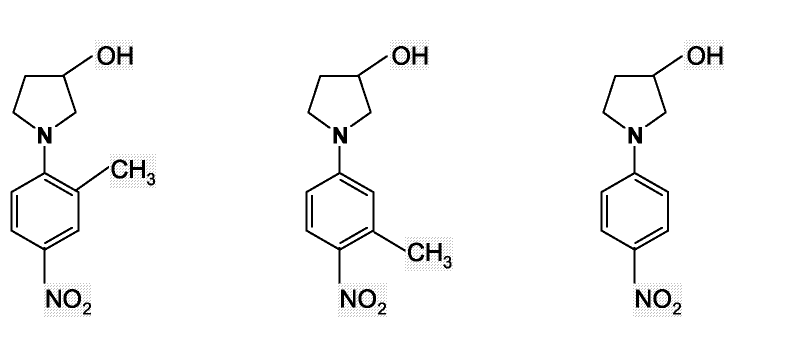

L'invention concerne enfin certains des composés de formule (XI) qui sont des composés nouveaux.

Il s'agit des composés de formule (XI) définis plus haut dans le cadre du procédé mais à l'exclusion de certains composés spécifiques et des composés où X=NO₂.

Ces composés nouveaux répondent à la formule (XI) : 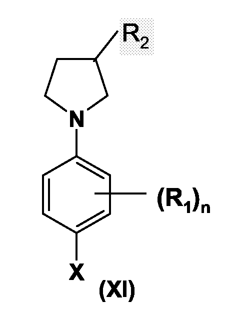 dans laquelle :
le carbone portant le substituant R₂ sur le cycle pyrrolidine est racémique ou chiral ; R₁, n, et X ont les mêmes significations que dans la formule (IX) ci-dessus sauf que X ne représente pas un groupement nitro ; et R₂ représente un radical azoté cationique ou non cationique ; excepté le composé de formule suivante :

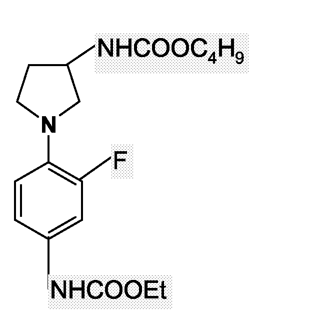

On pourra se reporter à ce qui a été indiqué auparavant concernant ces composés, notamment les définitions préférées des radicaux, de même que leur préparation.

L'invention va maintenant être décrite en référence aux exemples suivants, donnés à titre illustratif et non limitatif.

### Exemples

Dans les exemples 1 à 3 qui suivent, on prépare les composés (1), (2) et (3) pour le procédé selon l'invention.

### Exemple 1

Synthèse du chlorure de [1-(4-aminophényl)-pyrrolidin-3-yl]-triméthyl-ammonium, chlorhydrate (composé 1).

Le composé 1 est préparé selon le schéma suivant :

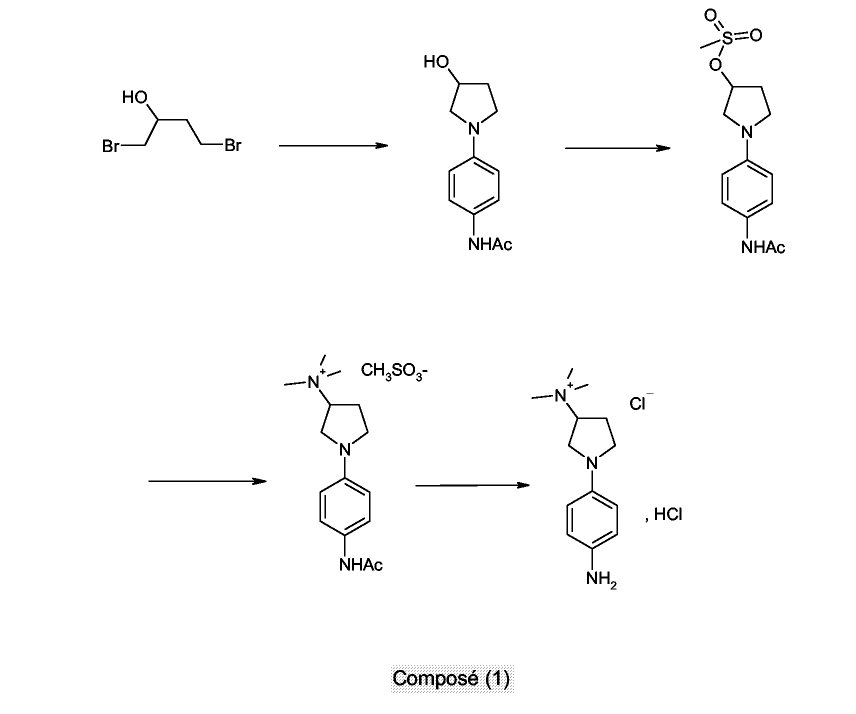

### Etape 1

N-[4-(3-hydroxy-pyrrolidin-1-yl)-phényl]-acétamide.

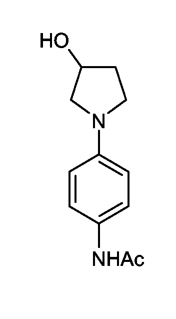

Dans un réacteur, sous atmosphère d'azote, on introduit 65 g (0,433 moles) de 4-amino-acétanilide et 68, 8 g (0, 649 moles) de Na₂CO₃ dans 260 ml d'isopropanol.

On porte au reflux puis on additionne goutte à goutte, 130,9 g de 1,4-dibromo-2-butanol. On chauffe au reflux pendant 4h puis on refroidit à 20°C.

On ajoute 260 ml d'eau, on filtre le précipité, on lave à l'eau puis on sèche sous vide à 40°C. On obtient 69,3g d'un solide beige soit un rendement final de 72,7%.

### Analyses

Les spectres de masse, et de RMN 1H et 13C sont conformes à la structure attendue.
Point de fusion (DSC) : 175,5°C

| Analyse élémentaire (C12H16N202 ; PM=220,27) | | | | |
|---|---|---|---|---|
| | C | H | N | O |
| % théorique | 65.44 | 7.32 | 12.72 | 14.53 |
| % trouvé | 65.43 | 7.37 | 12.85 | 14.78 |

### Etape 2

Ester de 1-(4-acétylamino-phényl)-pyrrolidine-3-yle de l'acide méthanesulfonique.

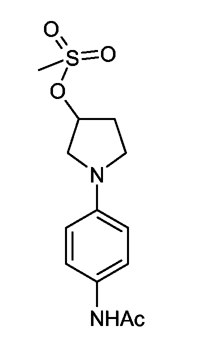

Dans un réacteur sous atmosphère d'azote, on introduit 50 g (0.227 moles) du composé de l'étape 1 dans 250 ml de pyridine. On refroidit à 0°C puis on ajoute 21,2 ml (0,272 moles) de chlorure de méthanesulfonyle en maintenant la température inférieure à 5°C. On agite à 5°C pendant 3h puis on verse le mélange dans 750 ml d'eau. On filtre, on rince le solide à l'eau puis on sèche sous vide à 40°C. On obtient 58 g d'un solide beige rosé soit un rendement final de 83%.

### Analyses

Les spectres de masse et de RMN 1H et 13C sont conformes à la structure attendue.
Point de fusion (DSC) : 150°-155°C

| Analyse élémentaire (C13H18N204S ; PM= 298,36) | | | | | |
|---|---|---|---|---|---|
| | C | H | N | O | S |
| % théorique | 52,33 | 6,08 | 9,39 | 21,45 | 10,75 |
| % trouvé | 52,21 | 6,08 | 9,36 | 21,50 | 10,62 |

### Etape 3

3-[1-(4-acétylamino-phényl)-pyrrolidin-3-yl]-triméthyl-ammonium, méthanesulfonate.

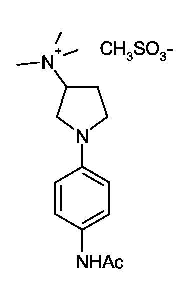

Dans un autoclave, on introduit 2 g (6,7 mmoles) du composé de l'étape 2 dans 20 ml d'une solution de triméthylamine dans l'éthanol (4,2M). On porte le mélange à 120°C pendant 4h puis on refroidit à 20°C. On coule le mélange sur 50 ml d'acétone, on filtre le précipité et on sèche sous vide. On obtient 1,4 g de produit brut lequel est recristallisé dans de l'isopropanol pour donner 1,25 g d'un solide beige clair soit un rendement final de 52%.

### Analyses

Les spectres de masse et de RMN 1H et 13C sont conformes à la structure attendue.

### Etape 4

Chlorure de [1-(4-aminophényl)-pyrrolidin-3-yl]-triméthyl-ammonium chlorhydrate (composé (1)).

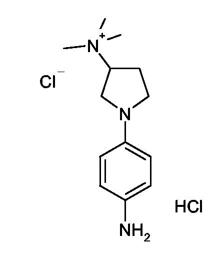

Dans un réacteur, on introduit 1 g du dérivé de l'étape 3, N-acétylé dans 5 ml d'éthanol et 1,2ml d'HCl concentré (35% aq.). On chauffe au reflux pendant 4h et on refroidit à 20°C. On filtre, on lave le précipité à l'isopropanol et on sèche sous vide sur potasse. On obtient 0,30 g d'un solide blanc, soit un rendement final de 36,5%.

### Analyses

Les spectres de masse et de RMN 1H et 13C sont conformes à la structure attendue.

### Exemple 2

Chlorure de [1-(4-aminophényl)-pyrrolidin-3-yl]-1-methyl-3H-imidazol-1-ium, chlorhydrate (composé (2)).

Le composé (2) est préparé selon le schéma suivant :

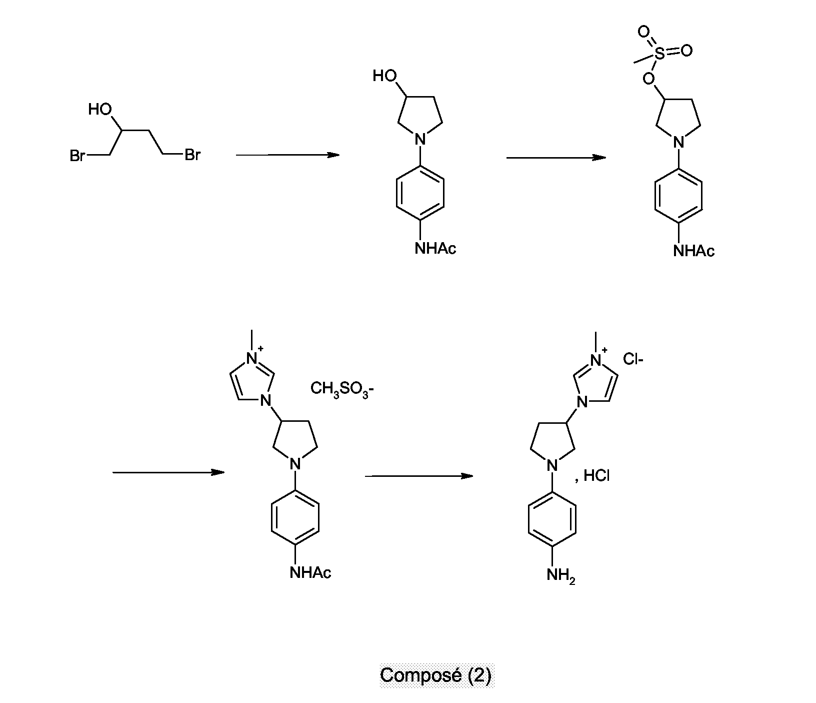

### Etape 1

Cette étape est analogue à l'étape 1 de l'exemple 1, dans laquelle on prépare le composé suivant :

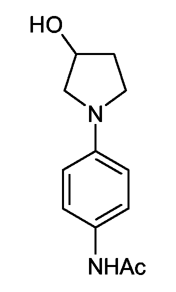

### Etape 2

Cette étape est analogue à l'étape 2 de l'exemple 1, dans laquelle on prépare le composé suivant :

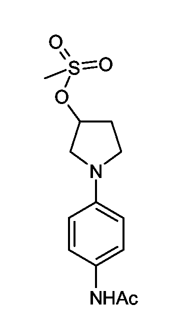

### Etape 3

3-[1-4-Acétylamino-phenyl)-pyrrolidine-3-yl]-1-méthyl-3H-imidazol-1-ium ; méthanesulfonate.

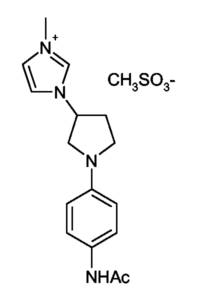

Dans un réacteur sous atmosphère d'azote, on introduit 37,5 g (0,1257 moles) du composé précédent de l'étape 2, dans 300 ml de méthylisobutylcétone. On porte le mélange à 80°C puis on ajoute goutte à goutte 40 ml de N-méthylimidazole. On chauffe ensuite au reflux à 120°C pendant 4h, on refroidit à 20°C puis on filtre. On lave le solide à l'acétone puis on sèche sous vide. On obtient 38 g d'un solide beige clair soit un rendement de 80%.

### Analyses

Les spectres de masse et de RMN 1H et 13C sont conformes à la structure attendue.

### Etape 4

Chlorure de [1-(4-aminophényl)-pyrrolidin-3-yl]-1-methyl-3H-imidazol-1-ium, chlorhydrate (composé (2)).

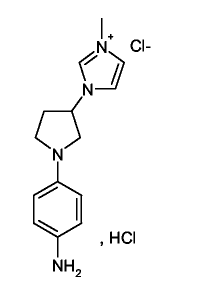

Dans un réacteur, on introduit 10 g du dérivé N-acétylé de l'exemple 3 dans 50 ml d'éthanol et 11 ml d'HCl concentré (37% aq.). On chauffe au reflux pendant 1H puis on élimine l'eau par distillation azéotropique. On refroidit à 20°C., on filtre, on lave le précipité à l'éthanol et on sèche sous vide sur potasse. On obtient 7,5 g d'un solide beige clair soit un rendement final de 91 %.

### Analyses

Les spectres de masse et de RMN 1H et 13C sont conformes à la structure attendue.
Point de fusion (DSC) : 287,2 °C

| Analyse élémentaire (C14H19N4Cl ClH ; PM=315,25 ) | | | | |
|---|---|---|---|---|
| | C | H | N | Cl |
| % théorique | 53,34 | 6,39 | 17,77 | 22,49 |
| % trouvé | 52,87 | 6,34 | 17,80 | 22,66 |

### Exemple 3

Dichlorhydrate de 1-(4-aminophenyl)-N,N-diméthylpyrrolidin-3-amine, (composé (3)).

Le composé (3) est préparé selon le schéma suivant :

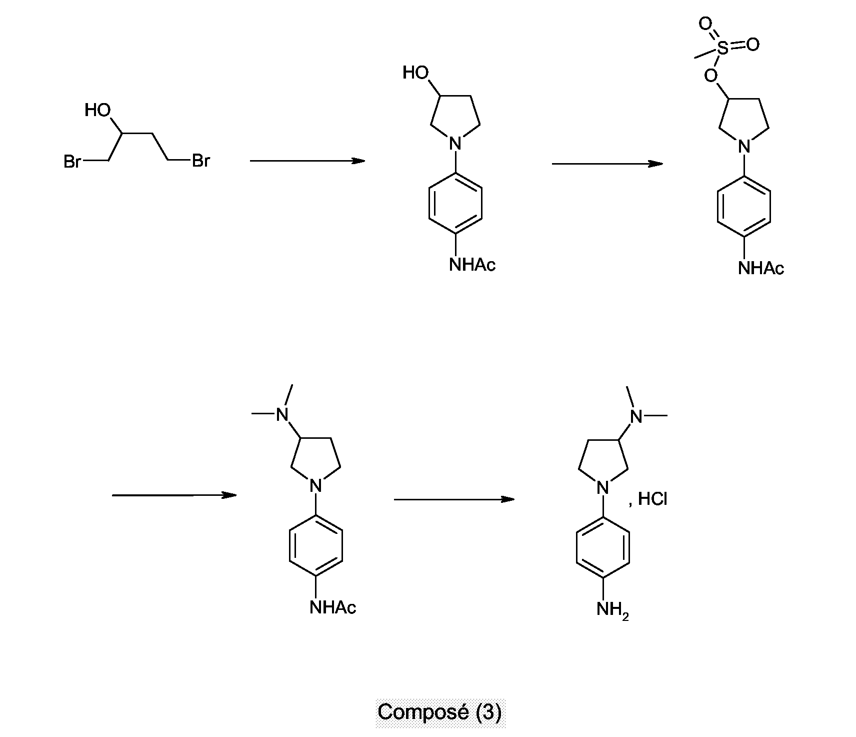

### Etapes 1 et 2

Ces étapes sont communes aux exemples 1 et 2.

### Etape 3

N-[4-(3-dimethylamino-pyrrolidin-1-yl)-phényl]-acétamide.

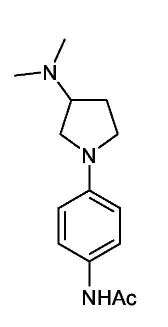

Cette étape est réalisée dans les mêmes conditions que dans l'exemple 1 et l'exemple 2, en utilisant comme groupement aminé, la diméthylamine en solution dans l'éthanol. On obtient 1,2 g d'un solide beige soit un rendement final de 70%.

### Analyses

Les spectres de masse et de RMN 1H et 13C sont conformes à la structure attendue.

### Etape 4

Dichlorhydrate de 1-(4-aminophenyl)-N,N-diméthylpyrrolidin-3-amine (composé 3).

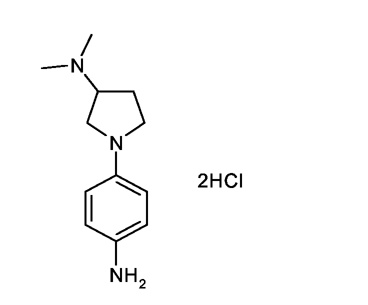

Selon les mêmes conditions opératoires que les exemples 1 et 2, à partir du composé N-acétylé précédemment décrit dans l'étape 3, on obtient le composé désiré avec un rendement final de 80%.

### Analyses

Les spectres de masse et de RMN 1H et 13C sont conformes à la structure attendue.

| Analyse élémentaire (C12H19N3, 2HCl ; PM=278,227) | | | | |
|---|---|---|---|---|
| | C | H | N | Cl |
| % théorique | 51,8 | 7,61 | 15,1 | 25,48 |
| % trouvé | 51,23 | 7,73 | 14,83 | 25,46 |

## Revendications

1. Procédé de synthèse de composés dérivés de paraphénylènediamine à groupement pyrolidinyle, substitués par un radical azoté, répondant à la formule
(I) suivante : 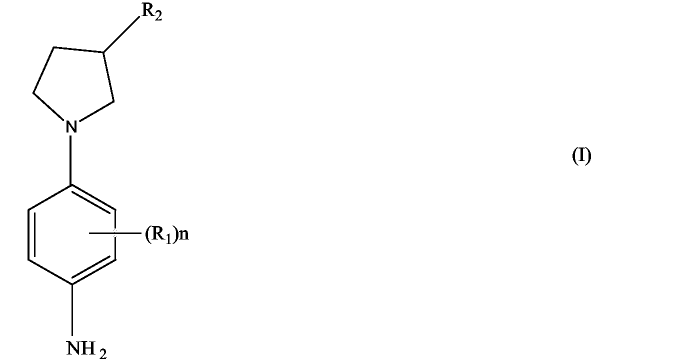
dans laquelle :
- le carbone portant le substituant R₂ sur le cycle pyrrolidine est racémique ou chiral ;
- n est un nombre entier de 0 à 4, étant entendu que lorsque n est supérieur ou égal à 2, alors les radicaux R₁ peuvent être identiques ou différents ;
- R₁ représente un atome d'halogène ; une chaîne hydrocarbonée aliphatique, linéaire ou ramifiée, en C₁-C₆, ou alicyclique en C₃-C₈, saturée ou insaturée, un radical aryle, un radical arylalkyle dont la chaîne alkyle est en C₁-C₆, un ou plusieurs atomes de carbone de la chaîne hydrocarbonée et de la chaîne alkyle du radical arylalkyle pouvant être remplacés par un atome d'oxygène, d'azote, de silicium, de soufre, ou par un groupement SO₂ ; le radical R₁ ne comportant pas de liaison peroxyde, ni de radicaux diazo, nitro ou nitroso ;
- R₂ représente un radical azoté cationique ou non ;
dans lequel on réalise les étapes successives suivantes :
a) on condense une aniline parasubstituée de formule (VII) : 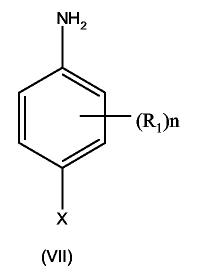 dans laquelle R₁ et n ont la signification déjà donnée plus haut pour la formule (I), et X représente un groupement précurseur d'une fonction amine, avec un dérivé de formule (VIII) : 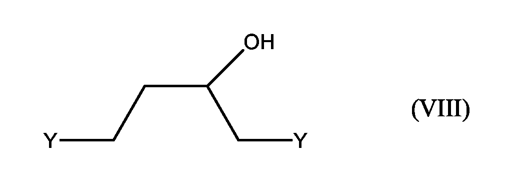 dans laquelle Y représente un groupe partant, pour former un composé de formule (IX) : 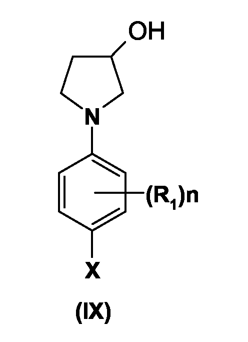 dans laquelle X, R₁ et n ont la signification déjà donnée plus haut ;
b) on active le composé de formule (IX) pour former un composé de formule (X) : 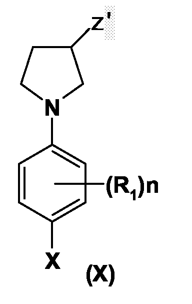 dans laquelle X, R₁ et n ont la signification déjà donnée plus haut et Z' représente un groupe partant ;
c) on fait réagir le composé de formule (X) avec :
- l'ammoniaque, ou une amine primaire, secondaire ou tertiaire,ou avec un composé portant un hétérocycle aromatique azoté ; pour former un composé de formule (XI) : 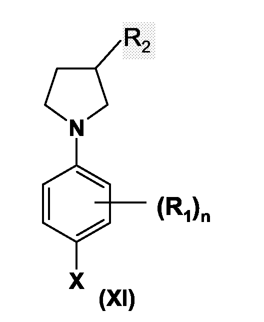
dans laquelle X, R₁, R₂ et n ont la signification déjà donnée plus haut ;
d) on transforme le groupement X en une fonction amine ; pour former le composé de formule (I).

2. Procédé selon la revendication 1, dans lequel n est égal à 0.

3. Procédé selon la revendication 1, dans lequel n est différent de zéro et par exemple égal à 1, et R₁ est un atome d'halogène ; une chaîne hydrocarbonée en C₁-C₆ aliphatique ou alicyclique en C₃-C₈, saturée ou insaturée, un radical aryle, un radical arylalkyle dont la chaîne alkyle est en C₁-C₆, un ou plusieurs atomes de carbone de la chaîne hydrocarbonée et de la chaîne alkyle du radical arylalkyle pouvant être remplacés par un atome d'oxygène, d'azote, de silicium, de soufre, ou par un groupement SO₂ ; le radical R₁ ne comportant pas de liaison peroxyde, ni de radicaux diazo, nitro ou nitroso.

4. Procédé selon l'une quelconque des revendications 1 et 3, dans lequel R₁ est choisi parmi le chlore, le brome, un radical alkyle en C₁-C₄, hydroxyalkyle en C₁-C₄, aminoalkyle en C₁-C₄ alcoxy en C₁-C₄, hydroxyalcoxy en C₁-C₄.

5. Procédé selon la revendication 4, dans lequel R₁ est choisi parmi un radical méthyle, hydroxyméthyle, 2-hydroxyéthyle, 1,2-dihydroxyéthyle, méthoxy, isopropyloxy, 2-hydroxyéthoxy.

6. Procédé selon l'une quelconque des revendications précédentes, dans lequel dans la formule
(I) R₂ représente un radical azoté non cationique choisi parmi un radical amine primaire (-NH₂), secondaire (-NHR) ou tertiaire (-NR₂) où R, identiques ou non, représentent un radical aliphatique, linéaire ou ramifié, saturé ou non, en C₁-C₂₂, de préférence un radical alkyle en C₁-C₂₂ ; un radical alicyclique, saturé ou non, en C₃_C₈ ; un radical monohydroxyalkyle en C₁-C₂₂, de préférence en C₁-C₆ ; un radical polyhydroxyalkyle en C₂_C₂₂, de préférence en C₂-C₆ ; un radical alcoxy (C₁-C₆) alkyle en C₁-C₂₂, de préférence en C₁-C₆ ; un radical aryle ; un radical arylalkyle dont la partie alkyle est en C₁-C₆, comme le radical benzyle par exemple ; un radical amidoalkyle en C₁-C₆ ; un radical trialkyl (C₁-C₆) silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl (C₁-C₆) carbonyle, amido ou alkyl (C₁-C₆) sulfonyle ; les radicaux R pouvant former deux à deux, ensemble avec l'atome d'azote auxquels ils sont rattachés, un cycle saturé carboné comprenant 3 à 9 chaînons, de préférence 4, 5, 6, 7 ou 8 chaînons, pouvant contenir un ou plusieurs hétéroatomes, tels les cycles azétidine, pyrrolidine, pipéridine, pipérazine, morpholine, ledit hétérocycle pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆, un radical amido, un radical carboxyle, un radical alkyl (C₁-C₆) carbonyle, un radical thio (-SH), un radical thioalkyle (-R-SH) en C₁-C₆, un radical alkyl(C₁-C₆)thio, un radical amino, un radical amino mono ou di-substitué par un radical alkyl(C₁-C₆), alkyl (C₁-C₆) carbonyle, amido ou alkyl (C₁-C₆) sulfonyle.

7. Procédé selon l'une quelconque des revendications 1 à 6, dans lequel dans la formule (I) R₂ représente un radical azoté non cationique dérivé de l'amino-guanidine.

8. Procédé selon l'une quelconque des revendications 1 à 5, dans lequel dans la formule (I) R₂ représente un radical onium correspondant à la formule (XIII) : 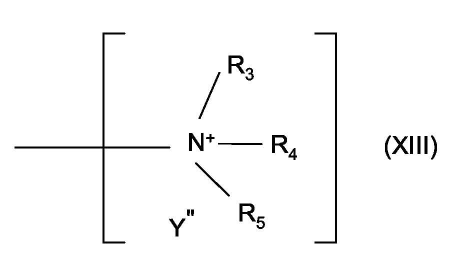 dans laquelle :
• R₃, R₄ et R₅, pris séparément, identiques ou non, représentent un atome d'hydrogène, un radical aliphatique, linéaire ou ramifié, saturé ou non, en C₁-C₂₂, de préférence un radical alkyle en C₁-C₂₂ ; un radical alicyclique, saturé ou non, en C₃-C₈ ; un radical monohydroxyalkyle en C₁-C₂₂, de préférence en C₁-C₆ ; un radical polyhydroxyalkyle en C₂-C₂₂, de préférence en C₂-C₆ ; un radical alcoxy (C₁-C₆) alkyle en C₁-C₂₂, de préférence en C₁-C₆ ; un radical aryle ; un radical arylalkyle dont la partie alkyle est en C₁-C₆, comme le radical benzyle par exemple ; un radical amidoalkyle en C₁-C₆ ; un radical trialkyl (C₁-C₆) silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl (C₁-C₆) carbonyle, amido ou alkyl (C₁-C₆) sulfonyle ;
• R₃, R₄ et R₅ ensemble, deux à deux, forment, avec l'atome d'azote auxquels ils sont rattachés, un cycle saturé carboné comprenant 3 à 9 chaînons, de préférence 4, 5, 6, 7 ou 8 chaînons, pouvant contenir un ou plusieurs hétéroatomes, tel que par exemple un cycle azétidine, un cycle pyrrolidine, un cycle pipéridine, un cycle pipérazine ou un cycle morpholine, le cycle pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆, un radical amido, un radical carboxyle, un radical alkyl(C₁-C₆)carbonyl, un radical thio (-SH), un radical thioalkyle (-R-SH) en C₁-C₆, un radical alkyl(C₁-C₆)thio, un radical amino, un radical amino mono ou di-substitué par un radical alkyl (C₁-C₆), alkyl (C₁-C₆) carbonyle, amido ou alkyl (C₁-C₆) sulfonyle ;
• Y" est un contre ion.

9. Procédé selon la revendication 8 dans lequel R₃, R₄ et R₅ séparément sont choisis parmi un radical alkyle en C₁-C₆, un radical monohydroxyalkyle en C₁-C₄, un radical polyhydroxyalkyle en C₂-C₄, un radical alcoxy (C₁-C₆) alkyle en C₁-C₄, un radical trialkyl (C₁-C₆) silanealkyle en C₁-C₆, ou R₃ avec R₄ forment ensemble un cycle azétidine, pyrrolidine, pipéridine, pipérazine, morpholine, R₅ étant choisi dans ce cas parmi un radical alkyle en C₁-C₆ ; un radical monohydroxyalkyle en C₁-C₆ ; un radical polyhydroxyalkyle en C₂-C₆.

10. Procédé selon la revendication 9, dans lequel dans la formule (I) R₂ est un trialkylammonium dont les radicaux alkyle peuvent être substitués.

11. Procédé selon l'une des revendications 1 à 5, dans lequel dans la formule (I) R₂ représente le radical onium correspondant à la formule (XIV) : 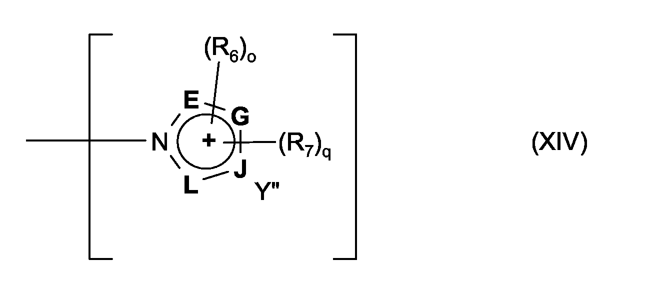 dans laquelle :
• les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique ;
• q est un nombre entier compris entre 0 et 4 inclus ;
• o est un nombre entier compris entre 0 et 3 inclus ;
• q+o est un nombre entier compris entre 0 et 4 inclus ;
• R₇, identiques ou différents, représentent un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆ ; étant entendu que les radicaux R₇ sont portés par un atome de carbone ;
• R₆ , identique ou différent, représente un radical alkyle en C₁-C₆, un radical monohydroxyalkyle en C₁-C₆, un radical polyhydroxyalkyle en C₂-C₆, un radical trialkyl (C₁-C₆) silanealkyle en C₁-C₆, un radical alcoxy (C₁-C₆) alkyle en C₁-C₆, un radical carbamylalkyle C₁-C₆, un radical alkyl (C₁-C₆) carboxyalkyle en C₁-C₆, un radical benzyle ; étant entendu que les radicaux R₆ sont portés par un azote ;
• Y" est un contre-ion.

12. Procédé selon la revendication 11 dans lequel les sommets E, G, J et L forment un cycle pyrrolique, imidazolique, pyrazolique, oxazolique, thiazolique ou triazolique.

13. Procédé selon la revendication 12 dans lequel les sommets E, G, J et L forment un cycle imidazolique.

14. Procédé selon l'une quelconque des revendications 1 à 5, dans lequel dans la formule (I) R₂ représente le radical onium correspondant à la formule (XV) : 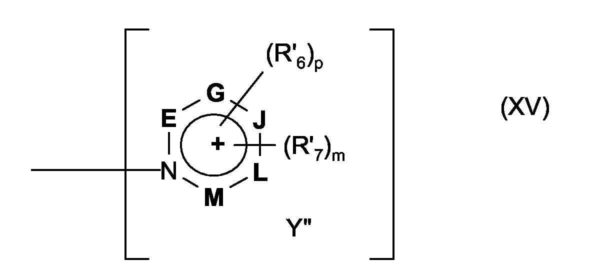 dans laquelle :
• les sommets E, G, J, L et M identiques ou différents, représentent un atome de carbone, d'oxygène, de soufre ou d'azote former un cycle choisi parmi les cycles pyridiniques, pyrimidiniques, pyraziniques, triaziniques et pyridaziniques ;
• p est un nombre entier compris entre 0 et 3 inclus ;
• m est un nombre entier compris entre 0 et 5 inclus ;
• p+m est un nombre entier compris entre 0 et 5 inclus ;
• R'₇, identiques ou différents, représentent un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆ ; étant entendu que les radicaux R'₇ sont portés par un atome de carbone ;
• R'₆, identique ou différent, représente un radical alkyle en C₁-C₆, un radical monohydroxyalkyle en C₁-C₆, un radical polyhydroxyalkyle en C₂-C₆, un radical trialkyl (C₁-C₆) silanealkyle en C₁-C₆, un radical alcoxy (C₁-C₆) alkyle en C₁-C₆, un radical carbamylalkyle C₁-C₆, un radical alkyl (C₁-C₆) carboxyalkyle en C₁-C₆, un radical benzyle ; étant entendu que les radicaux R'₆ sont portés par un azote ;
• Y" représente un contre-ion.

15. Procédé selon la revendication 14 dans lequel les sommets E, G, J, L et M forment avec l'azote du cycle un cycle choisi parmi les cycles pyridiniques, et pyrimidiniques.

16. Procédé selon l'une quelconque des revendications 11 à 15 dans lequel R₆, R₇ R'₇ et R'₆ sont des radicaux alkyles pouvant être substitués.

17. Procédé selon l'une quelconque des revendications précédentes dans lequel le composé de formule (I) est choisi parmi la liste ci-dessous.

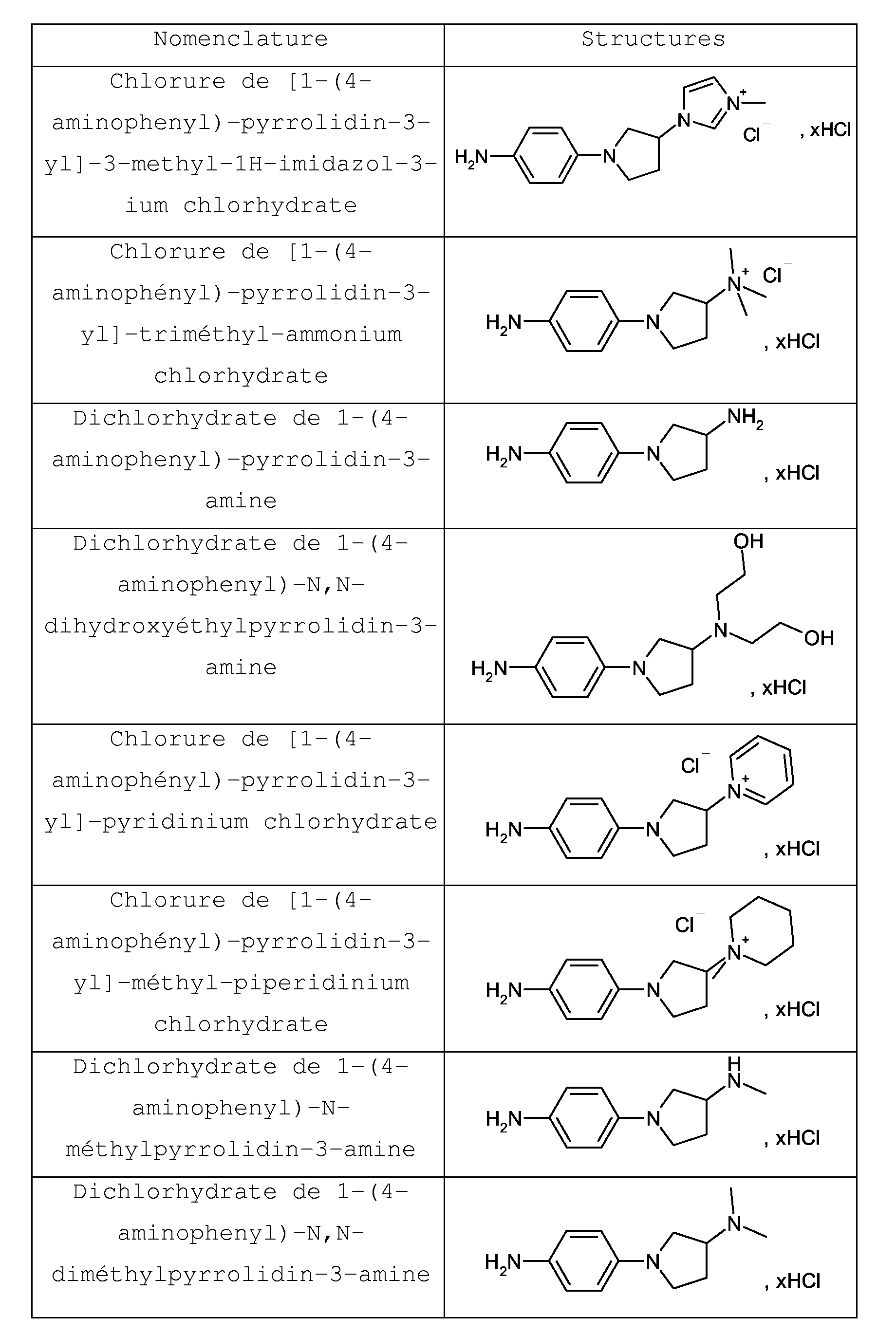

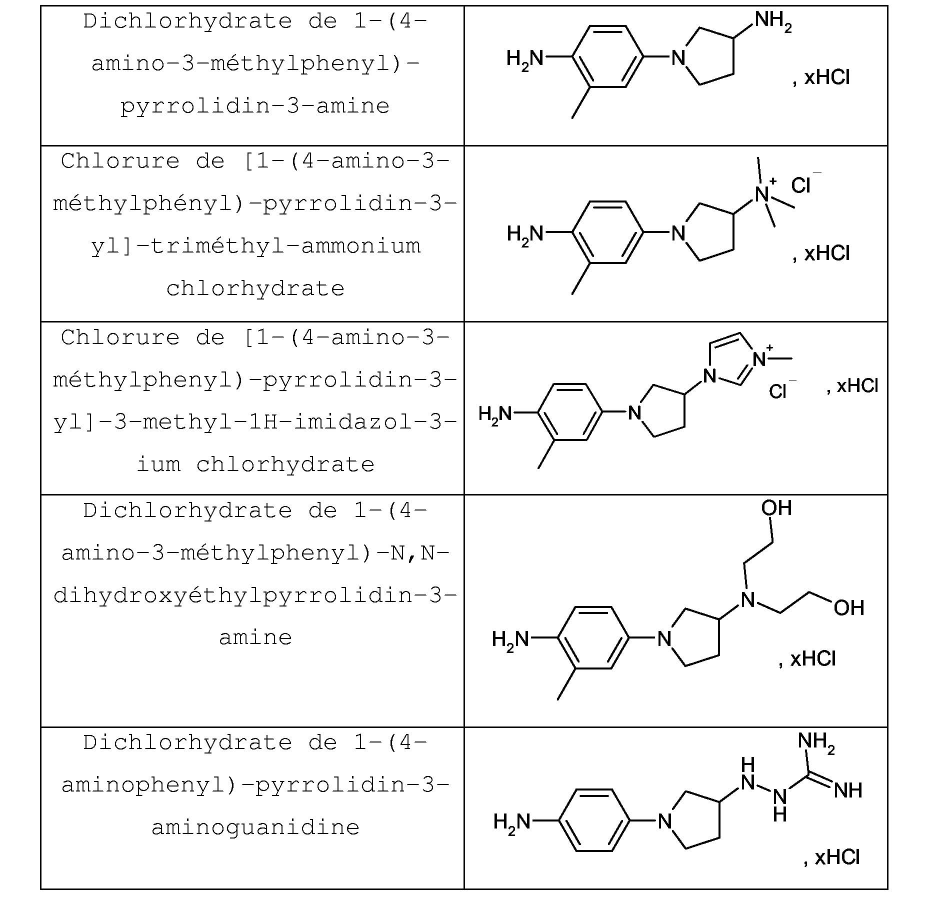

18. Procédé selon l'une quelconque des revendications précédentes, **caractérisé en ce que** l'étape d) consiste à mettre en oeuvre une étape de réduction telle que d'hydrogénation, ou d'hydrolyse.

19. Composés de formule (IX) suivante, ou leurs sels d'addition : 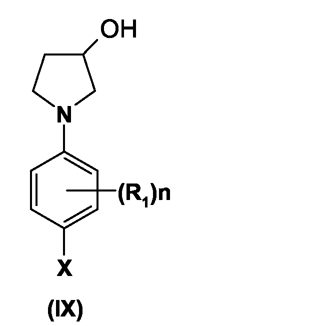 dans laquelle :
- le carbone portant le substituant hydroxyle sur le cycle pyrrolidine est racémique ou chiral ;
- n est un nombre entier de 0 à 4, étant entendu que lorsque n est supérieur ou égal à 2, alors les radicaux R₁ peuvent être identiques ou différents ;
- R₁ représente un atome d'halogène ; une chaîne hydrocarbonée aliphatique, linéaire ou ramifiée, en C₁-C₆, ou alicyclique en C₃-C₈, saturée ou insaturée, un radical aryle, un radical arylalkyle dont la chaîne alkyle est en C₁-C₆, un ou plusieurs atomes de carbone de la chaîne hydrocarbonée et de la chaîne alkyle du radical arylalkyle pouvant être remplacés par un atome d'oxygène, d'azote, de silicium, de soufre, ou par un groupement SO₂ ; le radical R₁ ne comportant pas de liaison peroxyde, ni de radicaux diazo, nitro ou nitroso ;
- X représente un groupement -NHCOR, -NHCOOR, un groupement nitroso ou un groupement nitro où R représentent une chaîne aliphatique, linéaire ou ramifiée, saturée ou non, de préférence saturée, en C₁-C₆, un groupement aryle ou un groupement arylalkyle dont la partie alkyle est en C₁-C₆ ; exceptés les trois composés dans le tableau ci-dessous : 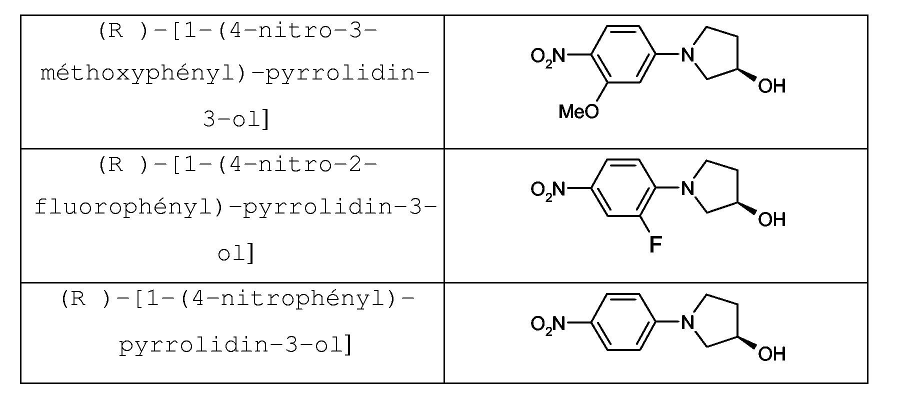 ; et exceptés les composés suivants :

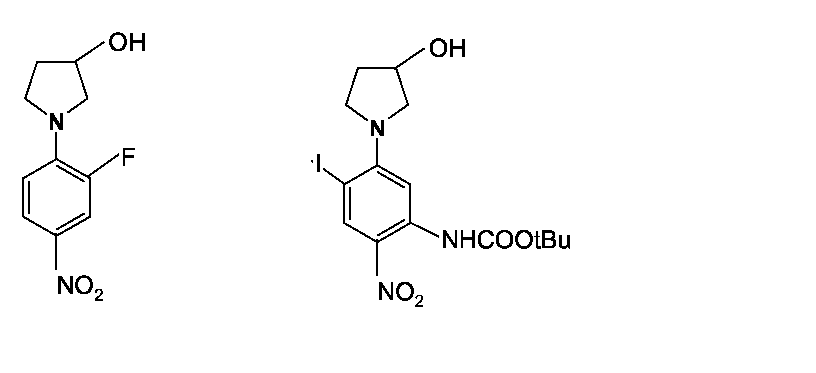

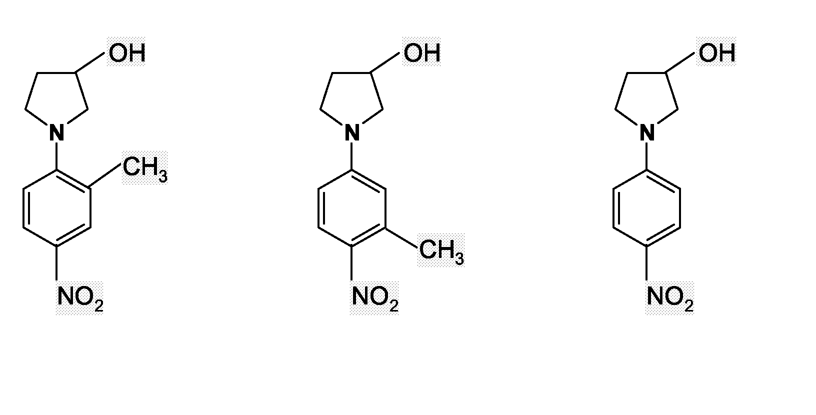

20. Composés selon la revendication 19, dans lesquels, dans la formule (IX), n est égal à 0.

21. Composés selon l'une des revendications 19 ou 20, dans lequel n est différent de zéro et par exemple égal à 1, et R₁ est un atome d'halogène ; une chaîne hydrocarbonée en C₁-C₆ aliphatique ou alicyclique en C₃-C₈, saturée ou insaturée, un radical aryle, un radical arylalkyle dont la chaîne alkyle est en C₁-C₆, un ou plusieurs atomes de carbone de la chaîne hydrocarbonée et de la chaîne alkyle du radical arylalkyle pouvant être remplacés par un atome d'oxygène, d'azote, de silicium, de soufre, ou par un groupement SO₂ ; le radical R₁ ne comportant pas de liaison peroxyde, ni de radicaux diazo, nitro ou nitroso.

22. Composés selon l'une quelconque des revendications 19 à 21, dans lesquels dans la formule (IX) R₁ est choisi parmi le chlore, le brome, un radical alkyle en C₁-C₄, hydroxyalkyle en C₁-C₄, aminoalkyle en C₁-C₄ alcoxy en C₁-C₄, hydroxyalcoxy en C₁-C₄.

23. Composés selon la revendication 22, dans lesquels dans la formule (IX), R₁ est choisi parmi un radical méthyle, hydroxyméthyle, 2-hydroxyéthyle, 1,2-dihydroxyéthyle, méthoxy, isopropyloxy, 2-hydroxyéthoxy.

24. Composés de formule (XI) suivante : 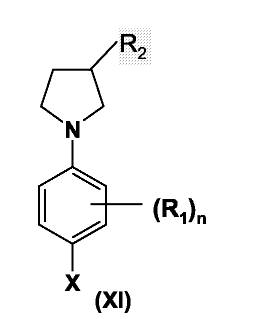 dans laquelle,
- le carbone portant le substituant R₂ sur le cycle pyrrolidine est racémique ou chiral ;
- n est un nombre entier de 0 à 4, étant entendu que lorsque n est supérieur ou égal à 2, alors les radicaux R₁ peuvent être identiques ou différents ;
- R₁ représente un atome d'halogène ; une chaîne hydrocarbonée aliphatique, linéaire ou ramifiée, en C₁-C₆, ou alicyclique en C₃-C₈, saturée ou insaturée, un radical aryle, un radical arylalkyle dont la chaîne alkyle est en C₁-C₆, un ou plusieurs atomes de carbone de la chaîne hydrocarbonée et de la chaîne alkyle du radical arylalkyle pouvant être remplacés par un atome d'oxygène, d'azote, de silicium, de soufre, ou par un groupement SO₂ ; le radical R₁ ne comportant pas de liaison peroxyde, ni de radicaux diazo, nitro ou nitroso ;
- R₂ représente un radical azoté cationique ou non ;
- X représente un groupement -NHCOR, -NHCOOR, ou un groupement nitroso, où R représentent une chaîne aliphatique, linéaire ou ramifiée, saturée ou non, de préférence saturée, en C₁-C₆, un groupement aryle ou un groupement arylalkyle dont la partie alkyle est en C₁-C₆ ;
excepté le composé de formule suivante :

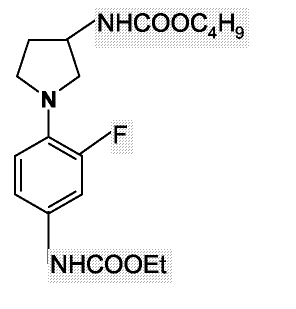

25. Composés selon la revendication 24, dans lesquels, dans la formule (XI), n est égal à 0.

26. Composés selon la revendication 24, dans lequel, dans la formule (XI), n est différent de 0 et est par exemple égal à 1, et R₁ est un atome d'halogène ; une chaîne hydrocarbonée en C₁-C₆ aliphatique ou alicyclique en C₃-C₈, saturée ou insaturée, un radical aryle, un radical arylalkyle dont la chaîne alkyle est en C₁-C₆, un ou plusieurs atomes de carbone de la chaîne hydrocarbonée et de la chaîne alkyle du radical arylalkyle pouvant être remplacés par un atome d'oxygène, d'azote, de silicium, de soufre, ou par un groupement SO₂ ; le radical R₁ ne comportant pas de liaison peroxyde, ni de radicaux diazo, nitro ou nitroso.

27. Composés selon l'une quelconque des revendications 24 et 26, dans lesquels, dans la formule (XI), R₁ est choisi parmi le chlore, le brome, un radical alkyle en C₁-C₄, hydroxyalkyle en C₁-C₄, aminoalkyle en C₁-C₄ alcoxy en C₁-C₄, hydroxyalcoxy en C₁-C₄.

28. Composés selon l'une quelconque des revendications 24 à 27, dans lesquels, dans la formule (XI), R₂ représente un radical azoté non cationique choisi parmi un radical amine primaire (-NH₂), secondaire (-NHR) ou tertiaire (-NR₂) où R, identiques ou non, représentent un radical aliphatique, linéaire ou ramifié, saturé ou non, en C₁-C₂₂, de préférence un radical alkyle en C₁-C₂₂ ; un radical alicyclique, saturé ou non, en C₃-C₈ ; un radical monohydroxyalkyle en C₁-C₂₂, de préférence en C₁-C₆ ; un radical polyhydroxyalkyle en C₂_C₂₂, de préférence en C₂-C₆ ; un radical alcoxy (C₁-C₆) alkyle en C₁-C₂₂, de préférence en C₁-C₆ ; un radical aryle ; un radical arylalkyle dont la partie alkyle est en C₁-C₆, comme le radical benzyle par exemple ; un radical amidoalkyle en C₁-C₆ ; un radical trialkyl (C₁-C₆) silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl (C₁-C₆) carbonyle, amido ou alkyl (C₁-C₆) sulfonyle ; les radicaux R pouvant former deux à deux, ensemble avec l'atome d'azote auxquels ils sont rattachés, un cycle saturé carboné comprenant 3 à 9 chaînons, de préférence 4, 5, 6, 7, ou 8 chaînons, pouvant contenir un ou plusieurs hétéroatomes, tels que les cycles azétidine, pyrrolidine, pipéridine, pipérazine, morpholine, ledit hétérocycle pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆, un radical amido, un radical carboxyle, un radical alkyl(C₁-C₆)carbonyl, un radical thio (-SH), un radical thioalkyle (-R-SH) en C₁-C₆, un radical alkyl (C₁-C₆) thio, un radical amino, un radical amino mono ou di-substitué par un radical alkyl (C₁-C₆), alkyl (C₁-C₆) carbonyle, amido ou alkyl (C₁-C₆)sulfonyle.

29. Composés selon l'une quelconque des revendications 24 à 27, dans lesquels, dans la formule (XI), R₂ représente un radical azoté non cationique dérivé de l'amino-guanidine.

30. Composés selon l'une quelconque des revendications 24 à 27, dans lesquels, dans la formule (XI), R₂ représente le radical onium correspondant à la formule (XIII) : 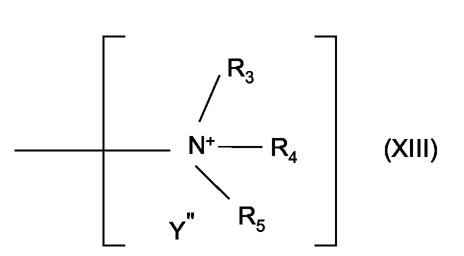 dans laquelle :
• R₃, R₄ et R₅, pris séparément, identiques ou non, représentent un atome d'hydrogène, un radical aliphatique, linéaire ou ramifié, saturé ou non, en C₁-C₂₂, de préférence un radical alkyle en C₁-C₂₂ ; un radical alicyclique, saturé ou non, en C₃-C₈ ; un radical monohydroxyalkyle en C₁-C₂₂, de préférence en C₁-C₆ ; un radical polyhydroxyalkyle en C₂-C₂₂, de préférence en C₂-C₆ ; un radical alcoxy (C₁-C₆) alkyle en C₁-C₂₂, de préférence en C₁-C₆ ; un radical aryle ; un radical arylalkyle dont la partie alkyle est en C₁-C₆, comme le radical benzyle par exemple ; un radical amidoalkyle en C₁-C₆ ; un radical trialkyl (C₁-C₆) silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl (C₁-C₆) carbonyle, amido ou alkyl (C₁-C₆) sulfonyle ;
• R₃, R₄ et R₅ ensemble, deux à deux, forment, avec l'atome d'azote auxquels ils sont rattachés, un cycle saturé carboné comprenant 3 à 9 chaînons, de préférence 4, 5, 6, 7 ou 8 chaînons, pouvant contenir un ou plusieurs hétéroatomes, tel que par exemple un cycle azétidine, un cycle pyrrolidine, un cycle pipéridine, un cycle pipérazine ou un cycle morpholine, le cycle cationique pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆, un radical amido, un radical carboxyle, un radical alkyl(C₁-C₆)carbonyl, un radical thio (-SH), un radical thioalkyle (-R-SH) en C₁-C₆, un radical alkyl(C₁-C₆)thio, un radical amino, un radical amino mono ou di-substitué par un radical alkyl (C₁-C₆), alkyl (C₁-C₆) carbonyle, amido ou alkyl (C₁-C₆) sulfonyle ;
• Y" est un contre ion.

31. Composés selon la revendication 30, dans lesquels, dans la formule (XI), R₂ est un trialkylammonium dont les radicaux alkyle peuvent être substitués.

32. Composés selon l'une quelconque des revendications 24 à 27, dans lesquels, dans la formule (XI),R₂ représente le radical onium correspondant à la formule (XIV) : 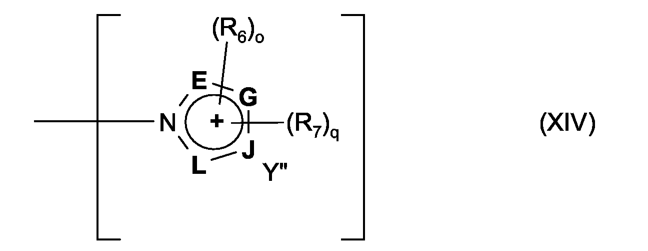 dans laquelle :
• les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique ;
• q est un nombre entier compris entre 0 et 4 inclus ;
• o est un nombre entier compris entre 0 et 3 inclus ;
• q+o est un nombre entier compris entre 0 et 4 inclus ;
• R₇, identiques ou différents, représentent un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆ ; étant entendu que les radicaux R₇ sont portés par un atome de carbone ;
• R₆ , identique ou différent, représente un radical alkyle en C₁-C₆, un radical monohydroxyalkyle en C₁-C₆, un radical polyhydroxyalkyle en C₂-C₆, un radical trialkyl (C₁-C₆) silanealkyle en C₁-C₆, un radical alcoxy (C₁-C₆) alkyle en C₁-C₆, un radical carbamylalkyle C₁-C₆, un radical alkyl (C₁-C₆) carboxyalkyle en C₁-C₆, un radical benzyle ; étant entendu que les radicaux R₆ sont portés par un azote ;
• Y" est un contre-ion.

33. Composés selon la revendication 32 dans lesquels les sommets E, G, J et L forment un cycle pyrrolique, imidazolique, pyrazolique, oxazolique, thiazolique ou triazolique.

34. Composés selon la revendication 33 dans lesquels les sommets E, G, J et L forment un cycle imidazolique.

35. Composés selon l'une quelconque des revendications 24 à 27, dans lesquels, dans la formule (XI), R₂ représente le radical onium correspondant à la formule (XV) : 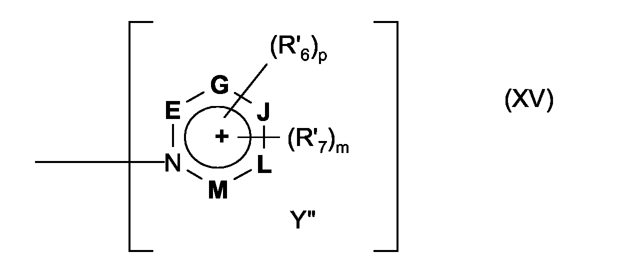 dans laquelle :
• les sommets E, G, J, L et M identiques ou différents, représentent un atome de carbone, d'oxygène, de soufre ou d'azote former un cycle choisi parmi les cycles pyridiniques, pyrimidiniques, pyraziniques, triaziniques et pyridaziniques ;
• p est un nombre entier compris entre 0 et 3 inclus ;
• m est un nombre entier compris entre 0 et 5 inclus ;
• p+m est un nombre entier compris entre 0 et 5 inclus ;
• R'₇, identiques ou différents, représentent un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆ ; étant entendu que les radicaux R'₇ sont portés par un atome de carbone ;
• R'₆, identique ou différent, représente un radical alkyle en C₁-C₆, un radical monohydroxyalkyle en C₁-C₆, un radical polyhydroxyalkyle en C₂-C₆, un radical trialkyl (C₁-C₆) silanealkyle en C₁-C₆, un radical alcoxy (C₁-C₆) alkyle en C₁-C₆, un radical carbamylalkyle C₁-C₆, un radical alkyl (C₁-C₆) carboxyalkyle en C₁-C₆, un radical benzyle ; étant entendu que les radicaux R'₆ sont portés par un azote ;
• Y" représente un contre-ion.

36. Composés selon la revendication 35 dans lesquels les sommets E, G, J, L et M avec l'azote du cycle forment un cycle choisi parmi les cycles pyridiniques, et pyrimidiniques.
